# EUROPÄISCHE PATENTSCHRIFT

(11) **EP 0 569 806 B1**
(45) Veröffentlichungstag und Bekanntmachung des Hinweises auf die Patenterteilung: **28.11.2001**
(21) Anmeldenummer: 93107120.3
(22) Anmeldetag: 03.05.1993
(51) Int. Cl.: C12N 15/54, C12N 15/81, C12N 9/10, C12P 25/00

(54) **DNA-Verbindungen und rekombinante, die Ribloflavinsynthetaseaktivität von S. cerevisiae codierende DNA-Expressionsvektoren**
DNA-compounds and recombinant DNA expression vectors coding for S. cerevisiae riboflavine synthetase activity
Composés ADN et vecteurs d'expression contenant ADN recombinant codant pour l'activité de riboflavine synthétase

(30) Priorität: 11.05.1992 ES 9200971
(43) Veröffentlichungstag der Anmeldung: 18.11.1993
(73) Patentinhaber: BASF Aktiengesellschaft, 67063 Ludwigshafen (DE)
(72) Erfinder: de los Angeles Santos Garcia, Maria, E-37009 Santa Marta (Salamanca) (ES); Garcia Ramirez, José Javier, E-26190 Nalda (La Rioja) (ES); Revuelta Doval, José Luis, E-37001 Salamanca (ES)

(56) Entgegenhaltungen:
- EP-A- 0 405 370
- WO-A-92/00379
- 'From genes to bioproducts' 1990 , DM PPU , MURICIA, SPANIEN BIOTEC 90 J.L. Revuelta et al. : Biosynthesis of vitamin B2 in yeast * Seite 117 - Seite 122; Abbildung 3 *
- YEAST (SPEC. ISSUE) Bd. 6 , 1990 Seite S330 SANTOS, M.A. ET AL. 'Molecular characterization of the riboflavin synthase-encoding gene, RIB5, from SACCHAROMYCES CEREVISIAE'
- 'Flavins and Flavoproteins' 1991 , WALTER DE GRUYTER , BERLIN, NEW YORK Proc. Int. Symp., 10th, 1990 Revuelta, J. L. et al.: Cloning and molecular characterization of the riboflavin synthase-encoding gene of Saccharomyces cerevisiae. * Seite 81 - Seite 84 *
- CURRENT GENETICS Bd. 14, Nr. 5 , November 1988 Seiten 419 - 423 MARIA DE LOS ANGELES SANTOS ET AL. 'Mapping of the RIB5 gene in SACCHAROMYCES CEREVISISAE using UV light as an enhancer of rad52-mediated chromosome loss'
- GENE Bd. 24, Nr. 1 , September 1983 , AMSTERDAM NL Seiten 1 - 14 MELLOR, J. ET AL. 'Efficient synthesis of enzymatically active calf chymosin in SACCHAROMYCES CEREVISIAE' & POUWELS, P.H. ET AL. 'Cloning vectors' 1987 , ELSEVIER , NL, AMSTERDAM
- GENE Bd. 61 , 1987 , AMSTERDAM NL Seiten 207 - 215 VERBAKEL J. M.A. ET AL. 'Construction of expression plasmids for SACCHAROMYCES CEREVISIAE: application for synthesis of poliovirus protein VP2' & POUWELS ET AL. 'Cloning vectors' 1987 , ELSEVIER , NL, AMSTERDAM
- NUCLEIC ACIDS RESEARCH Bd. 11, Nr. 9 , 1983 , ARLINGTON, VIRGINIA US Seiten 2745 - 2763 HITZEMAN, R. A. ET AL. 'Expression of hepatitis B virus surface antigen in yeast'
- BIOCHEMISTRY Bd. 9, Nr. 4 , 1970 , EASTON, PA US Seiten 771 - 785 PLAUT, G.W. E ET AL. 'Studies on the mechanism of elimination of protons from the methyl groups of 6,7-dimethyl-8-ribityllumazine by riboflavin synthetase'
- YEAST Bd. 9, Nr. 2 , Februar 1993 Seiten 189 - 199 DOIGNON, F. ET AL. 'The complete sequence of a 19,482 bp segment located on the right arm of chromosome II from SACCHAROMYCES CEREVISIAE' & PIR database accession number S34072 & EMBL database, entry SCCIIORF, accession number X70529

## Beschreibung

Vorliegende Erfindung betrifft DNA-Verbindungen und rekombinante DNA-Expressionsvektoren, welche die Expresssion der Riboflavinsynthetaseaktivität von Saccharomyces cerevisiae in rekombinanten Wirtszellen codieren und steuern, sowie in der Lage sind, die Vitamin B2 Ausbeute in Organismen zu verbessern, die dieses Vitamin herstellen.

Das Riboflavin oder Vitamin B2 ist ein Vorprodukt von Coenzymen, wie z.B. Flavin-Adenin-Dinukleotid (FAD) oder Flavinmononukleotid (FMN), die für die enzymatische Oxidation von Kohlehydraten erforderlich sind. Daher ist das Riboflavin unerläßlich für den grundlegenden Stoffwechsel bei allen Organismen. Pflanzen und eine Vielzahl von Mikroorganismen synthetisieren Riboflavin. Höhere Tiere stellen jedoch kein Riboflavin her. Unzureichende Riboflavinmengen können in höheren Tieren zu Haarausfall, Hautentzündungen, Sehschäden und Wachstumsstörungen führen.

Außerdem ist Riboflavin ein Nährstoff, der in Milch, Eiern, magerem Fleisch, Leber, Nieren, Gemüse, Getreide und insbesondere in Hefe enthalten ist, wobei Hefe die reichste natürliche Riboflavinquelle darstellt (The Merck, 9th Edition (1976), Windholz et al., eds, Merck & Co.,S.1064).

Riboflavin kann kommerziell durch rein chemische Synthese aus Ribose, wie in US 2.807.611 (Merck, 1957) beschrieben, oder durch Fermentation der Pilze Eremothecium ashbyii oder Ashbya gossypii (The Merck Index, supra) hergestellt werden. Außerdem können Riboflavinkonzentrate für die Ernährung von Geflügel und Rindern durch Fermentationsverfahren, wie in US 2.876.169 (Grain Processing Corp., 1959) beschrieben, hergestellt werden.

Die Herstellung von großen Mengen Riboflavin aus Mutanten von Bacillus subtilis wurde in folgenden Patenten beschrieben:
- US 3.900.386 (Enei et al, 1975). Dort werden Mutanten des Bacillus subtilis beschrieben, die durch Kontakt mit Azaguanin und Azaxanthin selektiert werden.
- FR 2.546.907 (Stepanov et al, 1984). Dort werden Mutanten von Bacillus subtilis beschrieben, die mit einem Plasmid (Riboflaviri-Operon) transformiert worden sind, das eine Kopie des Rib-Operons enthält. Grundlegende Verbesserungen der Eigenschaften dieses Operons wurden von Morozov et al. (Mol. Genet. Mik. Virusol. no 12:14 (1985)) und von Chikiudas et al. (Dikl. Akad. Nauk. 5, SSSR 298:997 (1988)) durchgeführt. Diese werden in die vorliegende Beschreibung als Bezug aufgenommen.

In der europäischen Patentanmeldung EP 0405370 (F. Hoffmann La Roche AG, 1991) werden Riboflavin überproduzierende Bakterienstämme von E.coli und B.subtilis beschrieben, welche eingebaute und erweiterte Kopien des S.cerevisiae Rib-Operons in ihrer Chromosomen-DNA enthalten.

Obwohl mit diesen Verfahren gute Ergebnisse erzielt werden können, haben einige Forscher versucht, aufgrund der geringen Genauigkeit bei der Expression von Proteinen in Bakterien sowie der Tatsache, daß die Bakterien nicht in der Lage sind, eine Glykosilierung der Proteine zu erreichen, Hefemutanten zu finden, die große Mengen an Riboflavin exprimieren. Somit beschreibt US 4.794.081 (Kawai et al, 1988), eine Riboflavin produzierende S. cerevisiae Mutante auf Purinbasis. Dieses Patent wird ebenfalls in die vorliegende Beschreibung als Bezug aufgenommen.

Revuelta et al. (Fram Genes to Bioproducts, 1990, DM PPU, Muricia, Spanien) offenbart die Klonierung des RIB 5 Gens, welches die Riboflavinsynthetaseaktivität von S. cerevisiae kodiert.

WO 92/00379 offenbart eine neue Promotor- und Terminatorregion aus dem Pilz Ashbya gossypii und deren Verwendung in Expressionssystemen, beispielsweise zur Expression der Gene der Vitamin B₂ Biosynthese.

In Gene, 1987, Bd 61, Seiten 207-215, wird die Verwendung des PGK Promotors zur Konstruktion eines Expressionsplasmids in S. cerevisiae beschrieben.

Vorliegende Erfindung betrifft die Verwendung des Riboflavinsynthetasegens aus Saccharomyces cerevisiae zur Herstellung von Expressionsvektoren für die Steigerung der Riboflavinsynthese in damit transformierten Wirtszellen.

Obwohl die für Riboflavinsynthetaseaktivität codierende DNA-Verbindung aus S.cerevisiae isoliert worden ist, kann diese CNA zur Herstellung von Vektoren verwendet werden, welche die Expression der Riboflavinsynthetaseaktivität in einer Vielzahl von Wirtszellen steuern. Da alle Riboflavin erzeugenden Organismen die gleiche Vorstufe verwenden, nämlich 6,6-Dimethyl-8-ribityllumazin, können die für Riboflavinsynthetaseaktivität codierenden DNA-Verbindungen zur Herstellung von anderen Vektoren verwendet werden, welche die Wirksamkeit und Ausbeute von Riboflavinfermentationen bei allen Riboflavin erzeugenden Organismen verbessern.

Die für Riboflavinsynthetaseaktivität codierenden DNA-Verbindungen wurden von genomischer DNA von S.cerevisiae abgeleitet und zusammen mit Aktivierungssequenzen der Transkription und Translation isoliert, welche die Expression der genomischen, die Riboflavinsynthetaseaktivität codierenden DNA kontrollieren.

Die DNA-Verbindungen, welche die Riboflavinsynthetaseaktivität von S. cerevisiae codieren, können sowohl (i) ausschließlich den codierenden Bereich umfassen, als auch (ii) diesen codierenden Bereich, zusammen mit Sequenzen oder Regulierungssignalen des Riboflavinsynthetasegens, die am 3'Ende des Codierstrangs im Codierbereich des Gens liegen. Diese regulierenden 3'-Sequenzen bestimmen das Ende der Transkription und der Polyadenylierung und RNA-Prozessierung des Riboflavinsynthetasegens von S. cerevisiae. Die Anwesenheit dieser Signale an der geigneten Position in einem Expressionsvektor (am 3'-Ende des Codierstrangs des zu exprimierenden Gens) erhöht die Expression des durch den Vektor codierten Produkts. Diese Sequenzen schließen alle Nukleotidsequenzen ein, die aufrund der Degeneration des genetischen Codes in der Lage sind, die Aminosäuresequenz der Riboflavinsynthetaseaktivität zu codieren, sowie alle Sequenzen, die genetische Varianten der DNA sind, welche die erfindungsgemäße Riboflavinsynthetaseaktivität codieren, wobei diese Varianten Äquivalente der DNA sein können, die gleiche oder eine ähnliche Aktivität besitzen können, und aufgrund der Homologie mit den genannten Verbindungen erhalten werden können.

Ein weiterer Gegenstand der Erfindung ist ein Verfahren zur rekombinanten Herstellung von Riboflavin durch Transformation einer Wirtszelle mit einem Expressionsvektor, der zur Expression der Riboflavinsynthetase von S. cerevisiae befähigt ist, dadurch gekennzeichnet, daß in dem geeigneten Expressionsvektor
a) eine, die Riboflavinsynthetaseaktivität codierende DNA-Verbindung, oder
b) eine zur obigen Verbindung homologe Sequenz, die diese Aktivität exprimiert und eine genetische Variante derselben darstellt, oder
c) ein die Riboflavinsynthetaseaktivität codierendes Fragment derselben,
kloniert wird.

Diese Gegenstände werden nachfolgend im Einzelnen beschrieben.
Figur 1 zeigt die Funktions- und Restriktionskarte des Plasmids YEp352 (1A) sowie die Subklonierung des Gens RIB5 (1B).
Figur 2 zeigt die Funktions- und Restriktionskarte des Plasmids pJR235.
Figur 3 zeigt den Aufbau des Plasmids pJR298 (3A), die Disruption des Gens RIB5 durch Gensubstitution (3B) und einen Southern blot (3C) der genomischen, mit Kpnl des wildwachsenden Stamms JC2a (MATαhis-Δ1 leu2-3 leu2-112 ura3-52) (Reihe 1) und zwei substituierten Stämmem AJ52 und AJ53 (MATαhis-Δ1 leu2-3 leu2-112 ura3-52 rib5-11) (RIB5::URA) (Reihen 2 und 3) fermentierten DNA. Der Filter wurde mit einer mit α-³²P markierten, vom Fragment Kpnl-Kpnl 3b abgeleiteten, Sonde hybridisiert.
Figur 4 zeigt den Aufbau der genetischen Fusion PGK-RIB5 durch rekombinantes PCR (4A) sowie die Funktions- und Restriktionskarte des Plasmids pJR376 (4B).

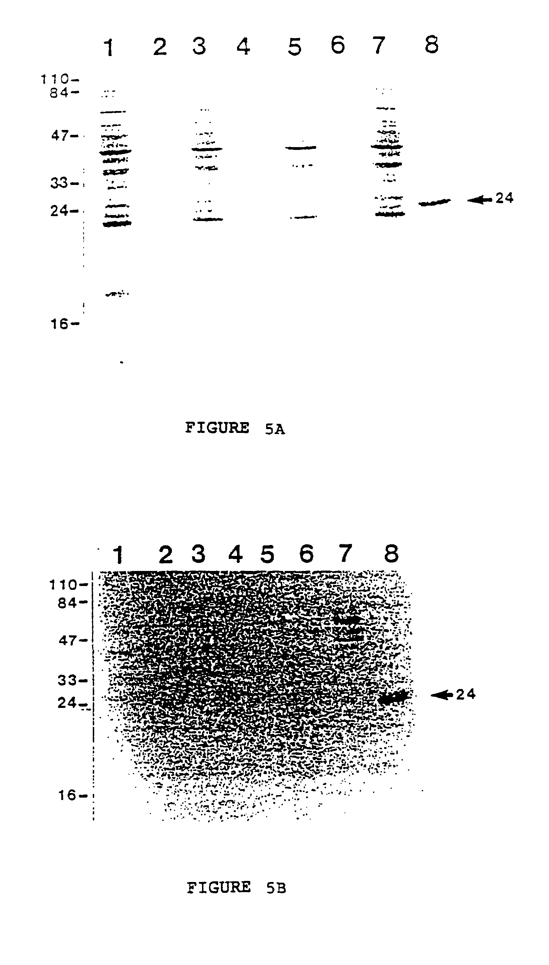

Figur 5 zeigt die Expression des Rib5-Proteins, der Riboflavinsynthetase, in E.coli Figur 5A, 14 %iges Polyacrylamid-SDS-Gel mit Coomasie Blau gefärbt, in dem die löslichen Fraktionen (Reihen 1, 3, 5 und 7) sowie die unlöslichen Fraktionen (Reihen 2, 4, 6 und 8) mit IPTG induzierten Zellextrakten (Reihen 3, 4, 7 und 8) oder nicht induzierten Zellextrakten (Reihen 1, 2, 5 und 6) der Stämme JM101/pTrc99-A (Reihen 1, 2, 3 und 4) sowie JM101/pJR380 (Reihen 5, 6, 7 und 8) analysiert worden sind. Figur 5B zeigt einen Western blot der löslichen und unlöslichen Fraktionen der analysierten Extrakte im Gel, das auf der Platte A vorgelegt wird, unter Verwendung von spezifischen Antikörpern gegen Rib5.

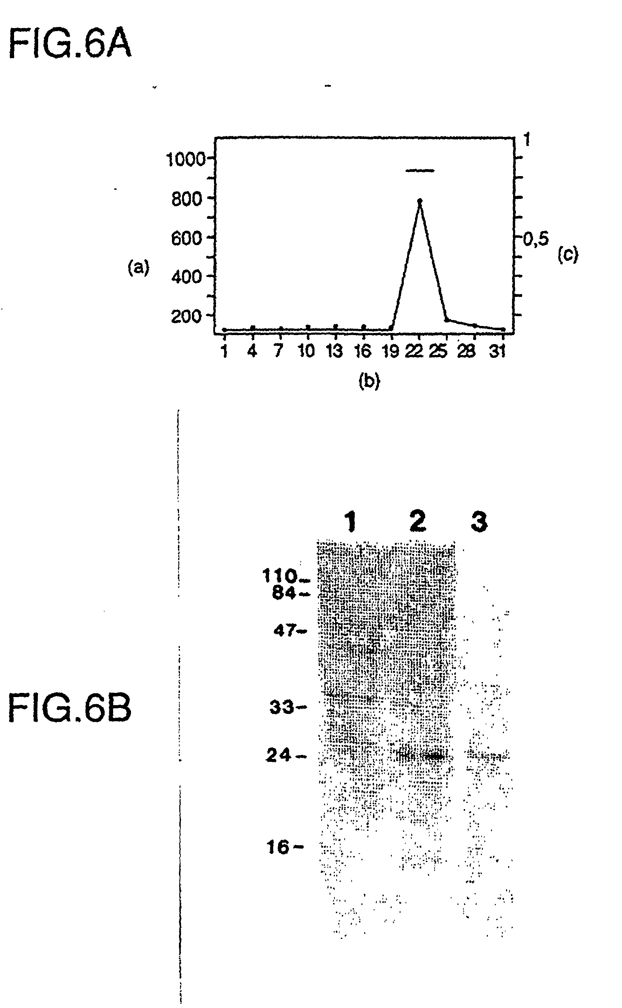

Figur 6 zeigt die Reinigung und Charakterisierung der Riboflavinsynthetase in S. cerevisae. Figur 6A zeigt die Reinigung durch FPLC aus einem Gesamtproteinextrakt in einer Ionenaustauschsäule (Mono Q HR5/5). Es wurde ein linearer NaCl-Gradient (diskontinuierliche Reihe) verwendet und verschiedene 1 ml Fraktionen, die im Hinblick auf die Anwesenheit des Rib5 Proteins durch Western blot (oberer Teil) und die spezifische Riboflavinsynthetaseaktivität (kontinuierliche Reihe) analysiert wurden. Figur 6B zeigt die Elektrophorese in einem SDS-Polyacrylamidgel: der Reihe 1, Gesamtproteinextrakt von S. cerevisiae; Reihe 2, durch FPLC gereinigte Riboflavinsynthetase, Reihe 3, Western blot der gereinigten Riboflavinsynthetase.
Figur 7 zeigt die Bestimmung des Molekulargewichts der Riboflavinsynthetase unter nativen Bedingungen.

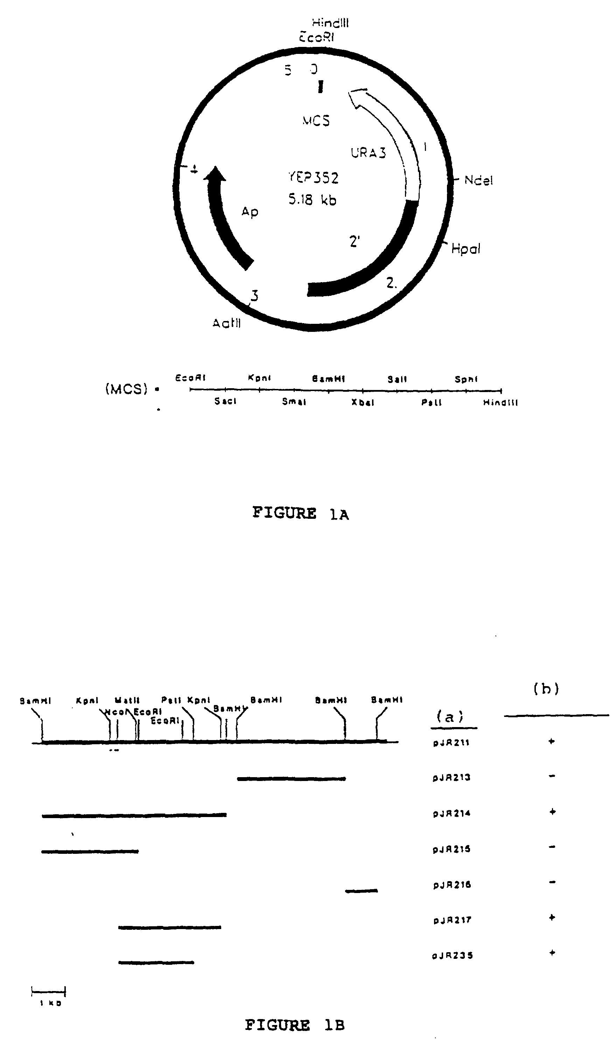

Die in den Figuren 1 bis 4 der beiliegenden Zeichnungen dargestellte Funktions- und Restriktionskarten sind annähernde Darstellungen der Vektoren der hier behandelten rekombinanten DNA. Die Ausdehnung der Restriktionsfragmente der Karte ist proportional zur Ausdehnung der Restriktionsfragmente der Vektoren, jedoch können die Abstände zwischen den Fragmenten etwas von den in den Karten errechneten Abständen abweichen. Die Hinweise zu den Restriktionsfragmenten sind nicht erschöpfend, so daß in einem bestimmten Vektor mehr Fragmente vorhanden sein können, als in der Karte dargestellt sind.

### A. Definitionen

Zur Verdeutlichung und zum besseren Verständnis der vorliegenden Erfindung, werden nachfolgend einige Begriffe definiert:

DNA-Verbindungen: DNA-Moleküle und DNA-Sequenzen, sowie deren Fragmente, welche alle zur Codierung eines genetischen Produkts erforderlichen Informationen erhalten, einschließlich die Transkription und Translation aktivierende Sequenzen derselben, sowie die Regulierungssignale der Expression der in dieser DNA enthaltenen Information. In der vorliegenden Erfindung wird stellenweise der Ausdruck "erfindungsgemäße DNA-Verbindungen" in dem Sinn verwendet, daß die DNA-Sequenz gemeint ist, welche die erfindungsgemäße Riboflavinsynthetaseaktivität codiert.

RIB5: DNA, welche die Riboflavinsynthetaseaktivität von Saccharomyces cerevisiae codiert.

URA3: DNA, welche die Orotidin-5'-phosphat-decarboxylase von Saccharomyces cerevisiae codiert.

Biosynthetisches Riboflavingen: ein DNA-Segment, welches die notwendige Aktivität für eine Reaktion im Umsetzungsverfahren der primären Stoffwechselprodukte in Riboflavin codiert.

Riboflavin herstellender Organismus: jeder Organismus, einschließlich aber nicht ausschließlich, Saccharomyces, Candida, Bacillus, Eremothecium und Ashbya, der Riboflavin herstellt, oder Gene enthält, die bei Expression Riboflavin herstellen.

ApR: Gen, das Resistenz gegenüber Ampicillin vermittelt.

Sac DNA: DNA von Saccharomyces cerevisiae.

Klonierung: Der Einbau eines DNA-Segments in einen Klonierungsvektor der rekombinanten DNA.

Genomische Genothek: Bündel von Klonierungsvektoren rekombinanter DNA, in dem DNA-Segmente geklont sind, die im wesentlichen das gesamte Genom des betreffenden Organismus darstellen. Hybridisierung: Paarungsverfahren zweier komplementärer, einkettiger DNA-Moleküle zu einem zweikettigen DNA-Molekül, das eine vollständigie oder parzielle Paarung von Basen sein kann. Riboflavinsynthetase: Ein Enzym, das die Riboflavinbildung aus 6,7-Dimethyl-8-ribityl-lumazin katalysiert.

Rekombinanter DNA-Expressionsvektor: Jedes autonome Replikations- oder Integrationsmittel, einschließlich (aber nicht ausschließlich) Plasmide, welches eine, die Transkription und/oder Translation aktivierende, Sequenz umfaßt, zur Steuerung der Expression eines DNA-Segments, das ein wissenschaftlich oder kommerziell interessantes Polypeptid oder ARN codiert. Restriktionsfragmente: Jedes geradkettige, durch Einwirkung einer oder mehrerer Restriktionenzyme erhaltene DNA-Molekül. Transformand: Eine empfangende Wirtszelle, die einer Transformation unterworfen wurde.

Transformation: Der Einbau der DNA in eine empfangende Wirtszelle, die den Erbtyp verändert und zu einer Änderung in der Empfängerzelle führt.

Aktivierungssequenz der Translation: Eine DNA-Sequenz, welche die DNA-Transkription fördert. Aktivierungssequenz der Translation: Eine DNA-Sequenz, die bei der Transkription zu ARNm die Translation von ARNm in Protein veranlaßt.

### B. DNA-Verbindungen, welche die Riboflavinsynthetaseaktivität von S.cerevisiae codieren.

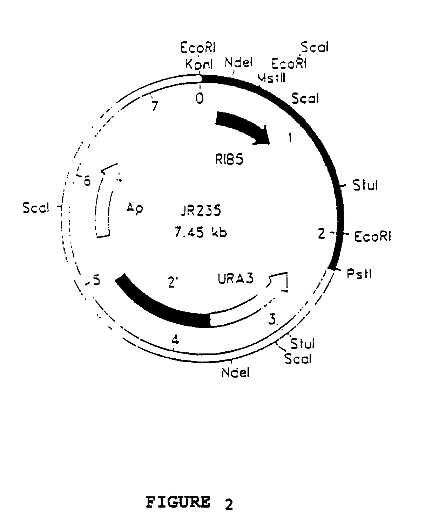

Zur Herstellung von DNA-Verbindungen, welche die Riboflavinsynthetaseaktivität von S. cerevisiae codieren, wurde eine genomische Genothek genomischer DNA von S. cerevisiae GRF88 im Centromervektor YCp50 (hinterlegt bei ATCC unter der Nr. 37415) verwendet, die zum Nachweis der Anwesenheit von Sequenzen untersucht wurde, die in der Lage. sind, die rib5-10 Mutation des Stamms AJ10 von S. cerevisiae durch Transformation gemäß Beispiel 2 zu komplementieren. Die rib5-10 Mutation, welche die die nicht funktionale Riboflavinsynthetaseaktivität codierende Sequenz beeinflußt, bestimmt die Synthese der nicht funktionalen Riboflavinsynthetase und führt zur Riboflavinauxotrophie und Akkumulation im 6,7-Dimethyl-8-ribityl-lumazinmedium in Trägerstämmen dieser Mutation. Eine Reihe von Vektoren in der genomischen Genothek enthielten eingebaute DNA-Fragmente von S. cerevisiae, die in der Lage waren, die rib5-10 Mutation zu komplementieren und das Wachstum von Transformationszellen des Trägerstamms AJ10 dieser Vektoren in einem riboflavinfreien Minimalmedium zu ermöglichen. Genetische Disruptions- und Sequenzierungsversuche gemäß Beispiel 2 ergaben, daß mindestens einer der Vektoren ein das Gen RIB5 tragende und die Riboflavinsynthetaseaktivität in S. cerevisiae codierende DNA-Fragment eingebaut enthielt. Das RIB5 Gen von S. cerevisiae wurde dann in einem anderen Vektor kloniert unter Bildung des Plasmids pJR235, das zur Transformation des JM101 Stamms von C. coli (Yanish-Perron et al., 1985, Gene 33:103) eingesetzt wurde. Die Transformanden von *E.coli* JM101/pJR235 wurden bei NCIMB unter der NCIMB-Nr. 40480 hinterlegt. Figur 2 zeigt eine Funktions- und Restriktionskarte des Plasmids pJR235.

Die codierende Sequenz des Riboflavinsynthetasegens von S. cerevisiae kann aus dem Restriktionsfragment KpnI-PstI 2.2 Kb des Plasmids pJR235 isoliert werden. Dieses Restriktionsfragment und die Codiersequenz wurden durch Sequenzierung der Nukleotide charakterisiert. Anschließend wird die die Riboflavinsynthetaseaktivität codierende DNA-Sequenz gezeigt zusammen mit einem DNA-Teil, welches das 3'-Ende des Codierbereichs im Genom von S. cerevisiae flankiert (SEQ ID NO 1). Es wird nur die Sequenz des codierenden oder "sense" Stranges des doppelsträngigen DNA-Moleküls gezeigt. Dies ist von links nach rechts in 5'→3' Richtung eingetragen. Bei der Numerierung der Nukleotidsequenzen erscheinen die Nummern über der DNA-Sequenz. Unmittelbar unter jeder DNA-Sequenzreihe werden von links nach rechts in aminoterminaler Richtung → terminales Carboxyl, die Aminosäurerestesequenzen der von der DNA codierten Riboflavinsynthetase gezeigt. Jeder Aminosäurerest erscheint unter dem Nukleotidtriplett, das ihn codiert. Bei der Numerierung der Sequenz der Aminosäurereste erscheint die Nummer unter der Sequenz der Aminosäurereste (SEQ ID NO 2).

Hier bedeuten: A Deoxyadenyl, G Deoxyguanyl, C Deoxycytidyl, T Thymidyl, ALA ein Alaninrest, ARG ein Argininrest, ASN ein Asparaginrest, ASP ein Asparginsäurerest, CYS ein Cysteinrest, GLN ein Glutaminrest, GLU ein Glutaminsäurerest, GLY ein Glycinrest, HIS ein Histidinrest, ILE ein Isoleucinrest, LEU ein Leucinrest, LYS ein Lysinrest, MET ein Metioninrest, PHE ein Phenylalaninrest, PRO ein Prolinrest, SER ein Serinrest, THR ein Threoninrest, TRP ein Tryptophanrest, TYR ein Tyrosinrest und VAL ein Valinrest.

Aufgrund der Degeneration des genetischen Codes, wodurch das Stoppsignal und die Mehrheit der Aminosäurereste durch mehr als ein Codon codiert sind, kann die oben gezeigte Aminosäuresequenz der Riboflavinsynthetase durch eine Vielzahl von DNA-Sequenzen codiert werden. Da diese alternativen DNA-Squenzen dieselbe erfindungsgemäße Aminosäuresequenz codieren, umfaßt die Erfindung ebenfalls diese alternativen Sequenzen.

Die Codiersequenzen der Riboflavinsynthetase können auf herkömmliche Weise oder durch die Phosphoramiditmethode unter Verwendung von vollständig geschützten Deoxyribonucleosiden synthetisiert werden. Diese Synthesemethoden sind gut bekannt und können nach dem Verfahren von Beaucage et al. (1981, Tetrahedron Letters 22:1859) durchgeführt werden. Die DNA-Sequenz kann mit automatischen DNA-Synthesizern, z.B von Applied Biosystems Model 380 A (Foster City, CA, USA) hergestellt werden.

Neben den oben beschriebenen DNA-Codiersequenzen der Riboflavinsynthetase können genetische Varianten der codierenden DNA-Sequenz der erfindungsgemäßen Riboflavinsynthetase vorkommen. Diese genetischen Varianten stellen die genetische Vielfalt der natürlich vorkommenden Formen dar und könnten eine wesentliche Homologie der Sequenz der Nukleotide und Aminosäurereste mit den erfindungsgemäßen Verbindungen aufzeigen. Diese könnten eine ähnliche, wenn nicht gar identische Aktivität haben, sich aber in irgendeiner Weise von der in der Beschreibung dargestellten Nukleotidsequenz unterscheiden. Diese genetischen Varienten sind Äquivalente der genannten Verbindungen und können aufgrund der Homologie mit den codierenden DNA-Sequenzen der erfindungsgemäßen Riboflavinsynthetase erhalten werden.

### B.1 Aktivierungssequenzen der Transkription und Translation

Die vorliegende Erfindung beruht auf der Klonierung einer intakten und funktionalen DNA-Sequenz, die nicht nur die Aminosäuresequenz der Riboflavinsynthetase codiert, sondern auch die für die Steuerung der Expression der Riboflavinsynthetase in S. cerevisiae erforderlichen Aktivierungssequenzen der Transkription und Translation. Gleichzeitig enthält das erfindungsgemäße Riboflavinsynthetasegen die dem Codierbereich nachfolgenden Sequenzen, die für die Termination der Transkription verantwortlich sind und die Prozessierungs- und Polyadenylierungssignale der DNA liefern. Diese Steuerelemente 5' und 3' sind ein wichtiger Aspekt der Erfindung. Da das pJR235 Gen ≃ 0.20 kb an genomischer DNA enthält, die im Genom von S. cerevisiae der Riboflavinsynthetase codierenden DNA vorangeht, enthält das pJR235 Plasmid zwangsläufig die Aktivierungssequenz für die Transkription und Translation des Riboflavinsynthetasegens von S. cerevisiae.

Die Mehrzahl der Aktivierungssequenzen der Transkription und Translation sind vor der zu aktivierenden DNA codiert, obwohl einige Sequenzen, welche die ribosomale RNA codieren durch Aktivierungssequenzen aktiviert werden, die nicht dem Codierbereich vorangehen. Dem Codierbereich "vorangehen" bezieht sich in diesem Zusammenhang auf die DNA in Richtung 5' des 5'-Endes des Codierstrangs der DNA, welche die Riboflavinsynthetase codiert. Die Aktivierungssequenzen der Transkription und Translation von S. cerevisiae, die im pJR235 Plasmid codiert sind, befinden sich an der richtigen Stelle, um die Expression der die Riboflavinsynthetase codierenden DNA zu steuern. Beim Aufbau von pJR235 wurden am 5'-Ende des Codierstrangs der die Riboflavinsynthetase codierenden DNA weder Deletionen noch Insertionen eingebaut, welche die Aktivierungssequenz der Transkription und Translation des Riboflavinsnthetasegens im Plasmid pJR235 beinflußen können, wobei diese Aktivierungssequenz in einem unmittelbar vorangehenden Restriktionsfragment KpnI-NdeI, das neben der die Riboflavinsynthetase codierenden DNA im pJR235 Plasmid liegt, isoliert werden kann. Die Stelle NdeI codiert die Aminosäuren 18 und 19 des Riboflavinsynthetaseproteins, so daß der Codierbereich des aminoterminalen Endes der Riboflavinsynthetase ebenfalls in diesem Fragment PpnI-NdeI enthalten ist. Jedes Restriktionsfragment, das das oben genannte Fragment von ≃ 0.20 kb KpnI-NdeI enthält, enthält zwangsläufig auch die erfindungsgemäße Aktivierungssequenz der Transkription und Translation.

Die DNA-Sequenz der im pJR235 codierten Aktivierungssequenz der Transkription und Translation von S. cerevisiae wird nachfolgend angegeben. Diese Sequenz kann chemisch synthetisiert werden, aus dem pJR235 Plasmid isoliert oder durch Polymerase-Kettenreaktion (PCR) aus dem pJR235 Plasmid oder der genomischen Gesamt-DNA von S. cerevisiae amplifiziert werden. Zur Klärung der Orientierung der Aktivierungssequenz im pJR235 Plasmid, wird das Restriktionsfragment als eine einkettige, sich überlappende DNA-Sequenz dargestellt, die für das Restriktionsenzym KpnI typisch ist und auch ein Initiationscodon der Translation einschließt.

### DNA-Sequenz von Saccaromyces cerevisiae

Im pJR235 Plasmid codierte Aktivierungssequenz der Transkription und Translation (SEQ ID NO 3)

Die Aktivierungssequenz der Transkription und Translation kann in jeder beliebigen DNA-Sequenz in S. cerevisiae oder in ähnlichen Organismen zur Steuerung der Expression eingesetzt werden. Der Transkriptionspromotor des Riboflavinsnthetasegens in S. cerevisiae kann an Codierbereiche verschiedener Proteinformen fusioniert werden. Die vor dem Initiationscodon der Transkription und Translation 5'-ATG liegende 5'-Sequenz kann z.B. zur Schaffung einer für die Restriktion geeigneten Stelle, wie z.B. Ndel oder Ncol, erweitert und modifiziert werden, durch Polymerase-Kettenreaktion (PCR) zur Fusion des Promotors von S. cerevisiae mit dem Codierbereich des interessierenden Proteins.

Der Codierbereich des Proteins kann auf ähnliche Weise so angepaßt werden, daß er zwei kompatible Enden enthält, die eine Kupplung ermöglichen. Hierzu werden je nach Codierbereich der Proteine unterschiedliche Strategien angewandt. Die Fusion des S. cerevisiae Promotors mit dem Codierbereich des interessierenden Proteins kann auch durch Polymerase-Kettenreaktion (PCR) nach der in Beispiel 3 dargelegten Methode erfolgen. In Beispiel 3 wird das Fusionsprotokoll des Codierbereichs der Riboflavinsynthetase von S, cerevisiae mit dem Transkriptionspromotor und der Aktivierungssequenz der Translation des PGK Gens von S. cerevisiae beschrieben.

Durch ähnliche Methoden kann der Codierbereich der Riboflavinsynthetase von S. cerevisiae mit Transkriptionspromotoren und Aktivierungssequenzen der Translation jedes beliebigen Organismus verknüpft werden.

Die vorliegende Erfindung ist nicht auf eine bestimmte Aktivierungssequenz der Transkription und Translation bei der Steuerung der Expression der die Riboflavinsynthetaseaktivität codierenden DNA beschränkt. Die Erfindung umfaßt die Verwendung beliebiger Aktivierungssequenzen der Transkription und Translation zur Expression der Riboflavinsynthetaseaktivität in S. cerevisiae.
Viele Aktivierungssequenzen der Transkription und Translation in S. cerevisiae sind bekannt und können zur Steuerung der Riboflavinsyn-thetaseaktivität in S. cerevisiae verwendet werden. Diese Aktivierungssequenzen der Transkription und Translation umfassen beispielsweise, aber nicht ausschließlich, Aktivierungssequenzen der Transkription und Translation von PGK, TPI, ADC1, ENO1, GAL1 und GAP. Neben den verschiedenen autonomen Replikationssequenzen und den oben beispielhaft genannten Aktivierungssequenzen der Transkription und Translation können autonome Replikationssequenzen und Aktivierungssequenzen der Transkription und Translation anderer Organismen mit der vorliegenden, die Riboflavinsynthetaseaktivität codierenden DNA-Verbindungen verknüpft werden, zur Bildung von Expressionsvektoren, welche die Expression der Riboflavinsynthetaseaktivität in Wirtszellen steuern, in denen die autonome Replikationssequenz und die Aktivierungsequenz tätig werden können. Obwohl S. cerevisiae ein geeigneter Wirt zur Herstellung der Riboflavinsynthetase für die Verwendung in vitro ist, können auch Expressionvektoren verwendet werden, welche die Expression der Riboflavinsynthetaseaktivität in anderen Wirtszellen als S. cerevisiae steuern, insbesondere dann, wenn die Wirksamkeit und Herstellungskapazität für Riboflavin in anderen Organismen gesteigert werden soll. Einige Riboflavin herstellende Organismen werden in der Industrie zur Herstellung von Vitamin B2 eingesetzt. Die Kapazität der Vitamin B2 Herstellung dieser Organismen kann durch Erhöhung der Konzentration der die Biosynthese limitierenden Enzyme während der Fermentation gesteigert und wirksamer gemacht werden.

### B.2 Regulierungssequenzen des Riboflavinsynthetasegens von S. cerevisiae.

Das pJR235-Plasmid umfaßt ebenfalls die 3'-Regulierungssequenzen des Riboflavinsynthetasegens von S. cerevisiae. Im allgemeinen sind die für die Termination der Transkription, Polyadenylierung und Prozessierung der mRNA verantwortlichen Sequenzen im Bereich ≃500 bp codiert, die dem Stoppcodon des Codierbereichs eines Gens folgen. Daher enthält das Scal-PstI-Fragment von ≃ 1.4 kb, welches die das carboxyterminale Ende der Riboflavinsynthetase codierende DNA umfaßt, auch die Terminationssignale der Transkription, Polyadenylierung und Prozessierung der mRNA des Riboflavinsynthetasegens von S. cerevisiae. Die Terminationssequenz der Transkription im pJR235 Plasmids kann an andere rekombinante Genkonstruktionen angefügt werden, um die Termination der Transkription in diesen genetischen Konstruktionen zu ermöglichen.

Die Expression einer gegebenen DNA-Sequenz in einem rekombinanten DNA-Expressionsvektor kann dadurch erhöht werden, daß ein Terminationssignal der Transkription, Polyadenylierung und RNAm-Prozessierung am 3' Ende des Codierstrangs des zu exprimierenden Codierbereichs eingefügt wird. Die vorliegende Erfindung liefert ein Terminationssignal der Transkription, Polyadenylierung und mRNA-Prozessierung, das zur Erhöhung der Expression in einem beliebigen genetischen Produkt eines rekombinanten DNA-Vektors in S. cerevisiae und verwandten Wirtsorganismen eingesetzt werden kann.

Die beschriebenen DNA-Verbindungen können zum Aufbau von Expressionsvektoren verwendet werden, welche die Expression der Riboflavinsynthetase in jeder beliebigen Wirtszelle steuern, in der der Expressionsvektor repliziert oder integriert wird und in der die Aktivierungssequenz der Transkription und Translation zur Expression der Riboflavinsynthetaseaktivität verwendet wird. Zusätzlich können die erfindungsgemäßen DNA-Verbindungen zur Codierung der Riboflavinsynthetaseaktivität von S. cerevisiae und zur Isolierung homologer DNA-Verbindungen anderer Stämme als S. cerevisiae verwendet werden, welche die genetischen Varianten der erfindungsgemäßen Riboflavinsynthetase codieren. Demzufolge umfaßt diese Erfindung auch homologe, die Riboflavinsynthetase codierende DNA-Verbindungen in den Plasmiden pJR235 und pJR376 (das im folgenden beschrieben wird), welche Riboflavinsynthetaseaktivität codieren.

### C. Rekombinante DNA-Expressionsvektoren, welche die Expression der Riboflavinsynthetaseaktivität von S. cerevisiae steuern.

Die für die Riboflavinsynthetaseaktivität von S. cerevisiae codierenden DNA-Verbindungen können zum Aufbau von Expressionsvektoren verwendet werden, welche diese Expression steuern. So kann z.B. das pJR235-Plasmid aus E. coli K12JM101/pJR235 nach dem in Beispiel 1 beschriebenen Verfahren isoliert werden. Aus diesem pJR235-Plasmid können sehr nützliche Restriktionsfragmente, wie z.B. ein KpnI-PstI von 2.2 kb erhalten werden, welches das gesamte RIB5-Gen enthält, oder ein NdeI-StuI-Fragment von 1.4 kb, welches das gesamte Gen, mit Ausnahme des aminoterminalen Endes, enthält. Das pJR235-Plasmid wurde als Basis zum Aufbau eines Plasmids mit der Bezeichnung pJR376 verwendet, welches in großem Umfang die Expression der Riboflavinsynthetaseaktivität in S. cerevisiae steuert. Das pJR376-Plasmid wurde erhalten durch Einfügung eines synthetischen DNA-Fragments von 2.2 kb,bestehend aus der Codiersequenz der Riboflavinsynthetase, unter Expression des Genpromotors des glycolischen Enzyms, 3-Phosphoglyceratkinase (PGK), von S. cerevisiae (Hitzeman et al., 1982, Nucleic Acids Res. 10:7791), in ein, dem YEp352-Plasmid ähnlichen Multikopienplasmid, das Sequenzen enthält, die eine autonome Replikation des Plasmids in *E. coli* und in S. cerevisiae ermöglichen, sowie das Ampicillinresistenz in E. *coli* vermittelnde β-Lactamasegen und das URA3-Gen von S. cerevisiae, das die Decarboxylase von Orotidin-5'phosphat codiert und die Selektion dieses auxotrophen Markers in *E. coli* und S. cerevisiae ermöglicht. Figur 1 der beiliegenden Darstellungen zeigt eine Funktions- und Restriktionskarte des YEp352-Plasmids. Im wesentlichen, ist das PGK-Gen eines der am wirksamsten in S. cerevisiae exprimierten Gene, so daß sowohl seine Transkripte, als auch das codierte Protein bis zu 5 % der Gesamttranskripte und der Zellproteine erreicht. Der PGK-Promotor kann so manipuliert werden, daß er hoch wirksame Expressionsvektoren verschiedener eukaryontischer Proteine produziert (Tuite et al., 1982, EMBO J. 1:603; Derynck et al., 1983, Nucleic Acid Res. 11:1819; Mellor et al., 1983, Gene 24:1; Wood et al., 1985, Nature (London) 314:446; Rothsteien et al., 1984, Nature (London) 308:662 und Adams et al., 1987, Nature (London) 329:68).

Das pJR376-Plasmid umfaßt den PGK-Promotor von der Position -580 bis -1 der Basenpaare, wobei die Numerierung mit dem Desoxyadenosylnukleotid des Initiationscodons 5'-ATG-3' der Translation als Position 1 beginnt, der die Expression der Codiersequenz des Riboflavinsynthetasegens des pJR235-Plasmids steuert. Die Konstruktion genetischer Fusionen durch Einsatz der Ketten-Polymerase-Reaktion (PCR) wird von Yon et al. (Nucleic Acids Res. 17:4895, 1989) beschrieben. Ein Aufbauprotokoll des Plasmids pJR376 ist im einzelnen in Beispiel 3 beschrieben.

Im SD-Ura-Medium wird die Riboflavinsynthetase in hohem Maße durch die das Plasmid pJR366 tragenden Zellen des Stamms S. cerevisiae in der Größenordnung von bis zu 2 % des gesamten Zellproteins exprimiert. Zellextrakte des S. cerevisiae transformierenden Stamms AJ53/pJ376 sind in der Lage, die Transformation von 6,7-Dimethyl-8-ribityl-lumazin in Riboflavin zu katalysieren, während die Zellextrakte des Stamms S. cerevisiae AJ53 diese Transformation nicht katalysieren können. Die Prüfmethode für diese Umsetzungsreaktion wird in Beispiel 4C beschrieben.

Das Plasmid pJR376 stellt einen wirksamen Mechanismus zur Herstellung großer Mengen Riboflavinsynthetase in S. cerevisiae zur Verfügung, und zwar in einem Umfang von ca. 1 % des gesamten Zellproteins. Aus diesem Grunde und weil die Kultivierung von S. cerevisiae weniger aufwendig ist, als die Kultivierung anderer Organismen, die Riboflavin natürlich produzieren, können S. cerevisiae/pJR376 Transformanden wirtschaftlicher und wirksamer als andere nicht rekombinante oder "natürliche" Riboflavinsynthetaseproduzenten zur Herstellung von Riboflavinsynthetase eingesetzt werden. Da die erfindungsgemäßen S. cerevisiae/pJR376 Transformanden in so großem Umfang Riboflavinsynthetase produzieren, kann die durch das Genom von S. cerevisiae codierte Riboflavinsynthetase in wesentlich reiner Form isoliert werden. Ein beispielhaftes Reinigungsprotokoll der Riboflavinsynthetase ist in Beispiel 4C dargestellt.

Wie bereits oben erwähnt, können die erfindungsgemäßen, die Riboflavinsynthetase codierenden DNA-Verbindungen zum Aufbau von Expressionsvektoren eingesetzt werden. Wenn mit diesen Riboflavinsynthetase-Expressionsvektoren eine Wirtszelle transformiert wird, die Riboflavin über eine durch Riboflavinsynthetase katalysierte Reaktion herstellt, so wird die Konzentration an Riboflavinsynthetase im Zellinnern erhöht.

Sofern die Riboflavinsynthetaseaktivität der begrenzende Faktor in der Riboflavinbiosynthese in der nicht transformierten Wirtszelle ist, werden die Wirtszellen, die diese Riboflavinsynthetase-Expressionsvektoren enthalten, mehr Riboflavin herstellen, als nicht transformierte Zellen.

Ein Vektor, der die Konzentration an Riboflavinsynthetase im Zellinnern einer durch ihn transformierten Wirtszelle erhöht, muß folgende Elemente enthalten: 1) eine DNA-Verbindung, welche die Riboflavinsynthetaseaktivität codiert; 2) eine Aktivierungssequenz der Transkription und Translation, die nicht nur in der zu transformierenden Zelle tätig werden kann, sondern auch die richtige Position und Ausrichtung hat, um die Expression der die Riboflavinsynthetase codierenden DNA zu steuern; 3) Replikationsund Integrationsfunktionen, die den Vektor in der Wirtszelle erhalten. Die Integrationsfrequenz eines Vektors oder eines DNA-Fragments hängt häufig von der durch die Wirtszellen codierten Aktivität ab. Es wird jedoch häufig festgestellt, daß die Anwesenheit bestimmter DNA-Sequenzen (z.B. die Integration fördernde Phagen- und Virensequenzen sowie homologe Sequenzen der genomischen DNA der Wirtszelle) in einem Vektor oder einem DNA-Fragment die Integration erleichtern.

Ein Riboflavinsynthetase-Expressionsvektor kann natürlich auch ein selektierbares Element enthalten, das eine Selektion der Wirtszellen ermöglicht, die diesen Vektor enthalten. Diese selektierbaren Elemente können jedoch unnötig oder unerwünscht sein, wenn der Vektor oder das DNA-Fragment in eine chromosomale DNA der Wirtszelle integriert wird.

Die Riboflavinsynthetase-Expressionsvektoren sind nicht auf einen bestimmten selektierbaren Marker beschränkt. Der Fachmann erkennt, daß hier viele selektierbare Marker bei der Verwendung von Riboflavinsynthetase-Expressionsvektoren geeignet sind. Diese selektierbaren Marker schließen auxotrophische Selektionsgene ein, z.B. das in leu2-Stämmen selektierbare LEU2-Gen (Hinnen et al. 1978, Proc. Natl. Acad. Sci. USA 75:1929), das in his2-Stämmen selektierbare HIS3-Gen (Struhl 1982, Nature 300:284) sowie das in *trpl*-Stämmen selektierbare TRP1-Gen (Hitzemann et al. 1980, J. Biol. Chem. 255:12073); Gene, die eine dominierende Selektion ermöglichen, wie z.B. das CUP1-Gen, das Kupferresistenz verleiht (Henderson et al. 1985, Curr. Genet. 9:133) sowie Gene die Antibiotikaresistenz verleihen, wie z.B. G418, den in Tn601 selektierbaren Marker (Jimenez and Davies, Nature, 287:869, 1980), Hygromycin, ein in dem Plasmid pIT123 selektierbarer Marker (Kaster er al. Curr. Genet. 8:353, 1983).

Einige Plasmide sind geeignet, die Konzentration an Riboflavinsynthetaseaktivität in einer Riboflavin herstellenden Zelle zu erhöhen. Das Plasmid pJR235 enthält das intakte Riboflavinsynthetasegen von S. cerevisiae, so daß die Transformation von S. cerevisiae mit dem Plasmid pJR235 eine Erhöhung der Kopienzahl des Riboflavinsynthetasegens erzeugt, was zu einer Erhöhung der Enzymkonzentration im Zellinnern führt.

Man geht davon aus, daß das Riboflavinsynthetasegen von S. cerevisiae in Ashbya gossypii und Eremothecium ashbii funktioniert. Daher führt die Transformation von A. gossypii und E. ashbyii über die Integration des Plasmids pJR235 zu einer Erhöhung der Kopienzahl des Riboflavinsynthetasegens, und somit zu einer Erhöhung der Enzymkonzentration im Zellinnern. Die erfindungsgemäße, die Riboflavinsynthetase von S. cerevisiae codierende Sequenz kann jedoch auch unter die Kontrolle von Aktivierungssequenzen der Transkription und Translation gestellt werden, die sich von Eremothecium und Ashbya ableiten, zum Aufbau eines rekombinanten, speziell für diese Organismen bestimmten Gens.

Die Expressionsvektoren von S. cerevisiae sind nicht auf die spezifischen hier beschriebenen Vektoren beschränkt. Die Erfindung umfaßt jedes beliebige Expressions-Plasmid von S. cerevisiae oder jeden Vektor, der die Expression der Riboflavinsynthetase in S. cerevisiae steuert. Daher umfaßt vorliegende Erfindung Riboflavinsynthetase-Expressionsvektoren, die eine funktionale Replikationseinheit in S. cerevisiae verwenden, wie z.B. die Replikationsursprungseinheit des Plasmids 2 µ und andere autonome Replikationssequenzen. Vorliegende Erfindung ist auch nicht beschränkt auf die Plasmid-Vektoren, da sie auch Expressionsvektoren oder DNA-Verbindungen umfaßt, welche die Riboflavinsynthetaseaktivität exprimieren und eine virale Replikation oder Integration im Genom verwenden, um, wie in Beispiel 3 beschrieben, in der Wirtszelle erhalten zu bleiben.

### D. Rekombinante, mit einen Expressionvektor, der eine die Riboflavinsynthetaseaktivität codierende DNA-Verbindung enthält, umgesetzte Mikroorganismen

Rekombinante Mikroorganismen, die mit einem Expressionsvektor umgesetzt worden sind, der eine für die Riboflavinsynthetaseaktivität codierende DNA-Verbindung enthält, können erhalten werden durch Einfügung eines erfindungsgemäßen Expressionsvektors in geeignete Wirtszellen und anschließende Kultivierung dieser Zellen unter Bedingungen, die für die Eingliederung in die chromosomale DNA der Wirtzelle oder seine Replikation in dieser Zelle geeignet sind, sowie Selektion der Transformanden. Geeignete Wirtszellen sind jeder Riboflavin herstellende oder nicht herstellende Mikroorganismus, wie z.B. Hefen, Bakterien oder Pilze. Vorzugsweise gehören diese Mikroorganismen zu den Gattungen Saccharomyces, Escherichia, Candida, Eremothecium und Ashbya, obwohl auch Mikroorganismen anderer Gattungen mit den Expressionsvektoren umgesetzt werden können. Die für die Transformation dieser Wirtszellen geeigneten Bedingungen hängen von der Art der zu transformierenden Zelle ab und sind dem Fachmann wohl bekannt. In diesem Zusammenhang wird Bezug genommen auf folgende Literatur: Maniatis et al., 1982, Molecular Cloning: A laboratory Manual, Cold Spring Harbor Laboratory Press, Cold Spring Harbor, New York und Yeast Genetics, Eds:J.F.T. Spencer, Dorothy M. Spencer und A.R.W. Smith, Springer-Verlag (1983), in der konventionelle Transformationstechniken für Mikroorganismen beschrieben werden.

In einer bevorzugten Ausführungsform werden die Transformanden von E. coli JM101/pJR235 hergestellt, die bei NCIMB unter der Nr. NCIMB 40480 hinterlegt worden sind.

### E. Herstellung der Riboflavinsynthetaseaktivität von S. cerevisiae

Ein weiterer wichtiger Gegenstand der Erfindung ist ein Verfahrenn zur Herstellung von S. cerevisiae Riboflavinsynthetaseaktivität. Im wesentlichen besteht dieses Verfahren darin, eine Kultur eines mit einem erfindungsgemäßen Expressionsvektor transformierten Organismus, der ein die Riboflavinsynthetase von S. cerevisiae codierende DNA-Verbindung enthält, anzulegen, wobei diese Kultur unter Bedingungen erfolgt, die für die Expression des eingebauten Gens geeignet sind. Diese Bedingungen hängen von der Art des transformierten Mikroorganismus ab und können im Bereich der bereits vorher genannten liegen.

Die Riboflavinsynthetetaseaktivität kann gereinigt werden oder ohne Reinigung direkt aus dem rohen Zellextrakt, das dieses Enzym enthält, verwendet werden (Beispiel 4).

Wie bereits erwähnt, stellt diese Erfindung eine Codiersequenz des S. cerevisiae Riboflavinsynthetasegens zur Verfügung, sowie eine Reihe von Expressionsvektoren, welche die Expression dieses Gens in S, cerevisiae steuern. Die Herstellung von Riboflavinsynthetase in S. cerevisiae ermöglicht hohe Expressionsraten und eine einfache Isolierung des Enzyms, so daß dieses zur Katalyse der in vitro-Kondensation neuer Lumazinderivate zur Herstellung neuer Flavinverbindungen eingesetzt werden kann. Die Transformation von *S.* cerevisiae, Candida guilliermondii, Candida flarerii, Eremothecium ashbyii, Ashbya gossypii und anderer Riboflavin herstellender Organismen mit. den erfindungsgemäßen, die Expression der Riboflavinsynthetase in der interessierenden Wirtszelle steuernden Expressionsvektoren führt zu einer höheren Riboflavinsynthetaserate und somit zu einem höheren Vitamingehalt in der transformierten Zelle.

Die Riboflavinsynthetase kann zur Herstellung von Riboflavin aus 6,7-Dimethyl-8-ribityl-lumazin in einem zellfreien System, wie in Beispiel 4C beschrieben, verwendet werden. Riboflavin ist nicht nur ein nützliches Vitamin, sondern auch ein Ausgangstoff für die Herstellung anderer wichtiger Cofaktoren, wie z.B. FMN und FAD. Ein weiteres wichtiges Einsatzgebiet der Riboflavinsynthetase ist die Kondensation von verschiedenen 6,7-Dimethyl-8-ribityl-lumazin-Verbindungen, die von der erfindungsgemäßen Riboflavinsynthetase erkannt werden können zur Herstellung von neuen Flavinderivaten.

### F. Herstellung von Riboflavin aus 6,7-Dimethyl-8-ribityl-lumazin

Ein weiterer wichtiger Gegenstand der Erfindung ist ein Verfahren zur Herstellung von Riboflavin aus 6,6-Dimethyl-8-ribityl-lumazin. Dieses Verfahren besteht im wesentlichen darin, eine 6,6-Di-methyl-8-ribityl-lumazin enthaltende Lösung mit der erfindungsgemäßen S. cerevisiae Riboflavinsynthetase zusammenzubringen. Diese Synthetase kann sowohl in gereinigeter Form, als auch, wie erwähnt, ungereinigt durch Verwendung eines rohen Zellextrakts, der diese Synthetase enthält, verwendet werden. Das Verfahren kann in Lösung oder durch Immobilisierung des Enzyms oder der Synthetase enthaltenden Zellen, in einem geeigneten Träger, durchgeführt werden.

Das Verfahren kann bei
- pH zwischen 6,5 und 7,5
- einer Temperatur zwischen 30°C und 40°C,
- mit einer Mindestkonzentration an Enzym von anfänglich 10 µg/ml Lösung durchgeführt werden.

Als feste Trägerstoffe, an die das gereinigte Enzym oder das Enzym aus einem rohen Zellextrakt gebunden werden kann, kommen die für diese Art Verfahren üblichen in Frage, z.B. Carraghenan, Sepharose (SIGMA) oder ein anderer geeigneter Träger.

In einer bevorzugten Ausführungsform der Erfindung wird das Riboflavin aus 6,7-Dimethyl-8-ribityl-lumazin in einem zellfreien System hergestellt (Beispiel 4).

### G. Herstellung neuer Flavine

Ein weiterer wichtiger Gegenstand der Erfindung ist ein Verfahren zur Herstellung neuer Flavine oder Flavinderivate unter Verwendung der erfindungsgemäßen Riboflavinsynthetase aus anderen Lumazinderivaten als das 6,7-Dimethyl-8-ribityl-lumazin, die von diesem Enzym als Substrat erkannt werden können. Die Riboflavinsynthetase kann in isolierter reiner Form oder in einem rohen Zellextrakt verwendet werden, und dieses Verfahren kann sowohl in Lösung, als auch durch Immobilisierung des reinem Enzyms oder der Zellen eines dieses Enzym enthaltenen Zellextrakts, in einem geeigneten festen Träger, in der oben beschriebenen Form durchgeführt werden.

Zellextrakte Riboflavin herstellender Organismen können zur Synthetisierung von nicht natürlichen (nicht in der Natur hergestellten) Flavinen verwendet werden. Die erfindungsgemäßen Expressionsvektoren liefern ein wirksames und preiswertes Verfahren zur Herstellung von Riboflavinsynthetase in rohen Zellextrakten, die zur in-vitro-Kondensation von nicht natürlich vorkommenden Lumazinen zur Herstellung von neuen Flavinen oder Flavinderivaten eingesetzt werden können. Die Suche nach nicht natürlichen Lumazinderivaten, die als Träger der Riboflavinsynthetase geeignet sind kann vervollständigt werden durch die Suche nach Riboflavinsynthetasemütanten, die nicht natürliche Lumazinderivate als Substrat akzeptieren. Vorliegende Erfindung liefert die Ausgangsstoffe für die Suche nach diesen Riboflavinsynthetasemutanten.

Vorliegende Erfindung wird anhand der nachfolgenden Beispiele veranschaulicht, die jedoch nur die vorliegende Erfindung verdeutlichen, nicht aber einschränken sollen.

### Beispiel 1

### Kultur von E. coli K12 JM101/pJR235 und Herstellung des Plasmids pJR235

### 1.A. Kultur von E. coli K12 JM101/pJR235

Ein E. coli K12 JM101/pJR235 Lyophilisat, das bei NCIMB unter der Nummer 40480 hinterlegt worden ist, kann als Impfkultur im nachfolgend beschriebenen Verfahren eingesetzt werden.

Ein Liter LB-medium (10 g Trypton, 10 g NaCl ung 5 g Hefeextrakt pro Liter), das 100 µm/1 Ampicillin enthält, wurde mit einer E. coli K12 JM101/pJR235 Impfkultur geimpft und unter Belüftung bei 37°C inkubiert, bis zu einer optischen Dichte bei 590 mn (O.D.₅₉₀) von ≃1. Dann wurden der Kultur 150 mg Chloramphenicol zugegeben und die Inkubation 16 Stunden lang fortgesetzt. Die Zugabe von Chloramphenicol verhindert die Proteinsynthese und somit spätere Zellteilungen, behindert aber nicht die Replikation des Plasmids.

### 1.B. Herstellung des Plasmids pJR235

Die nach Beispiel 1A hergestellte Kultur wurde 5 Minuten bei 4°C in einem Sorvall GSA Rotor (DuPont Co. Biotechnology Systems, Wilmington, DE 19880-0024) bei 6000 U/min zentrifugiert. Der entstandene Überstand wurde verworfen und der Zellniederschlag in 40 ml TES-Puffer (10 mM Tris-HCl, pH=7,5; 10 mM NaCl und 1 mM ADTA) gewaschen und gefällt. Der Überstand wurde wiederum verworfen und der Zellniederschlag in einem Trockeneis und Ethanol enthaltendem Bad gefroren und anschließend wieder aufgetaut. Der aufgetaute Zellniederschlag wurde erneut in 10 ml einer Lösung aus 25 % Saccharose und 50 mM EDTA suspendiert. Anschließend wurde der Lösung ca. 1 ml einer Lösung aus 5 mg/ml Lysozym, 3 ml EDTA 0,25 M, pH=8,0, sowie 100 µl RNAase A zugemischt, und diese wurde dann 15 min. in Eis inkubiert. Den mit Lysozym behandelten Zellen wurden 3 ml einer Lyselösung (hergestellt durch Vermischen von 3 ml 10 %-igem Triton-X 100, 75 ml EDTA 0,25 M, pH= 8,0, 15 ml Tris-HCl 1M, pH=8,0, und 7 ml Wasser) zugegeben, und nach dem Mischen wurde die entstandenen Lösung 15 min. in Eis inkubiert. Die lysierten Zellen wurden in einem Trockeneis-Ethanol-Bad gefroren und anschließend wieder aufgetaut.

Die Zellabfälle wurden von der Lösung durch 40 minütiges Zentrifugieren bei 25.000 U/min in einem SW27-Rotor (Beckman, 7360 Lincoln Ave., Lincolnwood, IL 60646) und durch Extraktion mit gepuffertem Phenol entfernt.

Ca. 30,44 g CsCl und ≃ 1 ml einer Lösung aus 5 mg/ml Ethidiumbromid wurden zu der vorgenannten Lösung gegeben, anschließend das Volumen auf 40 ml gebracht und in ein Rohr der Ultrazentrifuge Vti50 (Beckman) gegeben. Das Rohr wurde versiegelt und die Lösung in einem Rotor VTi50 bei 42.000 U/min 16 h zentrifugiert. Die mit UV-Licht sichtbar gemachte Querscheibe des entstandenen Plasmids wurde isoliert und in ein Rohr und Rotor Ti57 gegeben (die Volumenanpassungen wurden mit TES durchgeführt, das 0,761 g/ml CsCl enthielt) und 16 Stunden bei 50.000 U/min zentrifugiert. Die Plasmidquerscheibe wurde erneut isoliert, mit Salz gesättigtem Isopropanol zur Entfernung des Ethidiumbromids extrahiert und 1:3 mit TES-Puffer verdünnt. Anschließend wurden 2 Volumen Ethanol der Lösung zugegeben und diese dann über Nacht bei -20°C inkubiert. Die Plasmid-DNA wurde durch 15-minütiges Zentrifugieren der Lösung in einem Rotor SS34 (DuPont Co., Biotechnology Systems, Wilmington, DE 19880-0024) bei 10.000 U/min gefällt.

Die so erhaltene Plasmid-DNA pJR235 (≃ 1 mg) wurde in 1 ml TE-Puffer (10mM Tris-HCl, pH=8,0 und 1mM EDTA) resuspendiert und bei -20°C aufbewahrt.

In Figur 2 der beiliegenden Zeichnungen ist eine Funktions- und Restriktionskarte des Plasmids pJR235 dargestellt.

### Beispiel 2

### Klonierung und Analyse des Gens RIB5 von S. cerevisiae

Das allgemeine Verfahren zur Herstellung eines DNA-Fragments, das das funktionale, die Riboflavinsynthetase codierende RIB5-Gen von S. cerevisiae enthält, besteht in der Isolierung von Klonen aus einer genomischen Bibliothek von S. cerevisiae, die in der Lage sind, die rezessive rib5-10 Mutation funktional zu komplementieren. Die rib5 Mutationen beeinflussen das RIB5-Gen, welches die Riboflavinsynthetase in S. cerevisiae codiert und machen den Stamm, der diese Mutation enthält, unfähig, in einem riboflavinfreien Medium zu wachsen.

### 2.A. Beeinflußte rib5-Mutanten im die Riboflavinsynthetase codierenden Gen

Zur Steigerung der Frequenz der rib5 Mutationen wurde das Mutagen Ethylmethansulfonat (30µl/ml) (EMS; Sigma) eingesetzt. Die Mutagenese wurde in Zellen des Stamms SI502 von S. cerevisiae (MATa his-3-Δ1 leu2-3 leu2-112 ura3-52) nach den üblichen Verfahren durchgeführt (Lawrence, C.W. Guide to Yeast Genetics and Molecular Biology, Methods Enzymol. 194:273, 1991). Nach der Saat von 2 x 10⁵ mutagenisierten Zellen im mit Riboflavin ergänztem (20 µg/ml) YPD-Medium und anschließender Replikation im vollständigem Synthesemedium ohne Riboflavin (SC-rib) wurden die auxotrophen Mutanten, die nicht fähig sind, im riboflavinfreien Medium zu wachsen, nachgewiesen und selektiert (Sherman, F. Guide to Yeast Genetics and Molecular Biology, Methods Enzymol. 194:1, 1991). Die rib5-Mutanten wurden unter den für Riboflavin auxotrophen Mutanten anhand ihrer Fähigkeit identifiziert, 6,7-Dimethyl-8-ribitil-lumazin im Kulturmedium anzuhäufen (Oltmanns et al., Mol.Gen. Genet, 105:306, 1969). Zur Klonierung des RIB5-Gens wurde der Mutant AJ10 (MATa his-3-Δ1 leu2-3 leu2-112 rib5-10 ura3-52) ausgewählt.

### 2.B. Isolierung des das RIB5-Gen enthaltenden Plasmids pJR211

Zur Isolierung von Plasmiden, die das RIB5-Gen tragen und in der Lage sind, die rib5-10-Mutation des Stamms AJ10 von S. cerevisiae zu vervollsändigen, wurde eine DNA aus einer genomischen Bibliothek von S. cerevisiae GRF88 im centromeren Plasmid YCp50 verwendet, die von ATCC unter der ATCC-Nr. 37415 erhalten wurde. Konkret wurden kompetente, durch Behandlung mit Lithiumacetat erhaltene Zellen des Stamms AJ10 von S. cerevisiae mit 65 µg DNA der Genothek in YCp50 analog dem in Beispiel 1B beschriebenen Verfahren inkubiert. Die Reaktionsmischung wurde in ein vollständiges Synthesemedium gesäht, in dem weder Riboflavin noch Uracil enthalten war, um eine doppelte Selektion des im Vektor YCp50 enthaltenen Markers URA3 und des im Plasmid der Genothek enthaltenen und zu isolierenden RIB5-Markers durchzuführen. Von insgesamt 13.000 geschätzten Transformanden (Phenotyp Ura+) wurden 3 Klone erhalten, die in einem uracil- und riboflavinfreien Medium (Phenotyp Ura+ Rib+) wachsen können. Die Plasmide, deren Träger diese drei Transformanden waren, wurden durch Transformation in Bakterien zurückgewonnen und mit Restriktionsenzymen charakterisiert (Strathern and Higgins, Guide to Yeast Genetics and Molecular Biology, Methods Enzymol., 194:319, 1991). Die drei Plasmide haben identische Restriktionsmuster und eines von ihnen, das pJR211, wurde für weitere Analysen ausgewählt. Das Plasmid pJR211, das aus dem Vektor YCp50 und einem eingebauten DNA-Fragment von ≃ 13 kb besteht, ist in der Lage, beim Einbau in den Stamm AJ10 in gleicher Effizienz die rib5-10 und ura3-52-Mutation zu vervollständigen.

### 2.C. Subklonierung des RIB5-Gens und Aufbau des Plasmids pJR235

Zur Abgrenzung des kleinsten, das RIB5-Gen enthaltenden DNA-Fragments, wurde eine Karte für eine Reihe von Restriktionsenzymen des Fragments ≃ 13 kb des Plasmids pJR211 erstellt und verschiedene Unterfragmente isoliert und an den episomalen Vektor YEp352 gebunden. Die entstandenen Plasmide wurden nach ihrer Fähigkeit, die rib5-Mutation zu vervollständigen, geprüft und aus ihnen ging hervor, daß das RIB5-Gen im Fragment KpnI-PstI ≃ 2.2 kb enthalten war. Eine Funktions- und Restriktionskarte des Vektors YEp352 und der für die Subklonierung eingesetzten Fragmente ist in Figur 1 der beiliegenden Zeichnungen dargestellt. Das Fragment KpnI-PstI wurde im Vektor YEp352 geklont, unter Bildung des Plasmids pJR235, dessen Restriktions- und Funktionskarte in Figur 2 der beiliegenden Zeichnungen dargestellt ist.

### 2.D. Aufbau eines Stamm mit vollständiger Deletion des Gens RIB5

Zum Aufbau eines S. cerevisiae-Stamms mit einer stabilen genetischen Deletion des größten Teils des Codierbereichs des RIB5-Gens wurde die Methode der einstufigen genetischen Disruption (Rothstein, R. Recombinant DNA, Part C, Methods Enzymol., 101:202, 1983) angewandt. Die Methode basiert auf der Fähigkeit zur Rekombination der DNA-Moleküle mit freien Enden, die während der Umsetzung von Hefen durch direkte Interaktion mit homologen Sequenzen im Genom die Rekombination anregen. Auf diese Weise wird im interessierenden Gen eine Disruption in vitro aufgebaut, so daß die Sequenzen des Gens durch die eines selektierbaren Marker ersetzt werden (z.B. URA3). Die in vitro erzeugte Disruption wird später zur Ersetzung der genomischen Sequenzen während der Umsetzung verwendet, so wie es in Figur 3B skizziert ist. Ein Beispiel dieses Aufbaus wird im folgenden beschrieben.

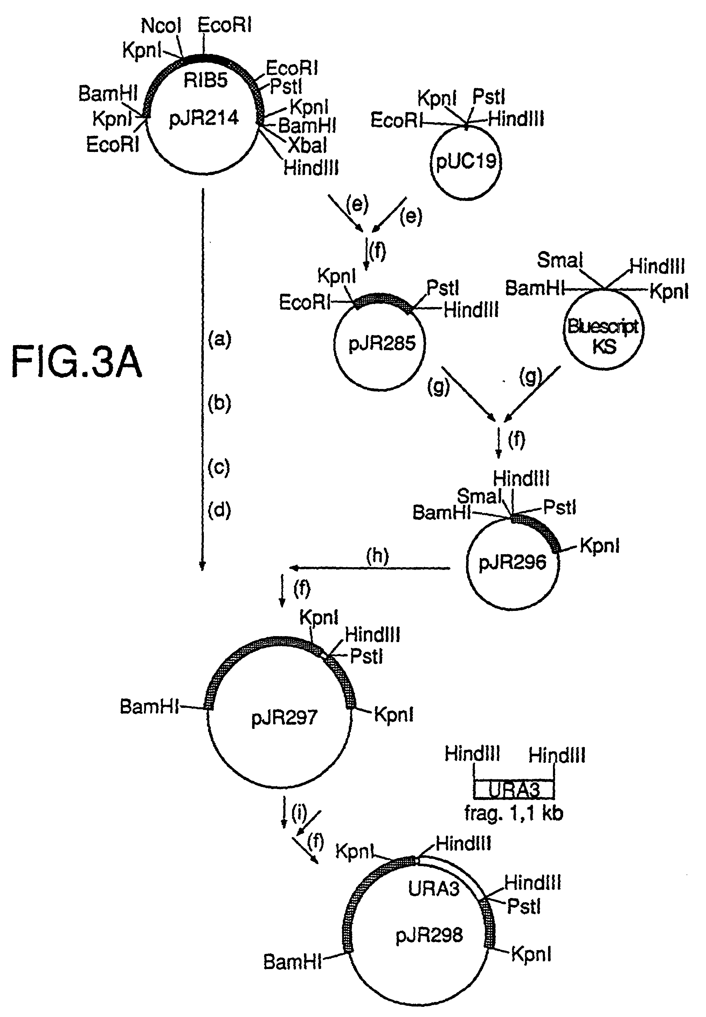

Wie Figur 3A zeigt, wurde das Plasmid pJR214 zunächst mit Ncol verdaut, die Enden durch Behandlung mit dem Klenowfragment der Polymerase I glatt gemacht, anschließend mit BamHI und dem Fragment BamHI-Ncol ≃ 2kb digeriert, welches das 5' Ende des RIB5-Gens flankiert, in Agarosegel gereinigt, an den Bluescript KS+ Vektor ligiert (Stratagene, La Jolla, CA, USA), mit BamHI und SmaI digeriert und in E. coli JM101 transformiert. Ein rekombinantes Plasmid wurde selektiert und pJR278 genannt. Auf ähnliche Weise wurde das Fragment KpnI-PstI ≃ 0,8 kb des Plasmids pJR214, welches das 3' Ende des RIB5-Gens flankiert, zunächst in pUC19 subkloniert (Yanisch-Perron et al. Gene 33:103, 1985; zu erhalten in New England Biolabs, Boston, MA USA und Bethesda Research Laboratories, Maryland, USA), von diesem als Fragment HindIII-KpnI ≃ 0,8 kb befreit, an pJR278 ligiert, das vorher mit HindIII und KpnI verdaut worden war, und in E. coli JM101 transformiert.

Ein rekombinantes Plasmid wurde selektiert und pJR297 genannt. Schließlich wurde das HindIII-HindIII-Fragment ≃ 1,1 kb, welches das funktionale URA3-Gen von S. cerevisiae enthält (Botstein et al, Gene, 8:17, 1979), in pJR297 kloniert, unter Bildung des pJR298-Plasmids. Der Verdau von pJR298 mit KpnI setzt das Fragment von ≃ 1,9 frei, bestehend aus dem URA3-Gen, das flankiert wird von das 5' und 3' Ende des RIB5-Gens flankierenden Sequenzen. Dieses Fragment wurde gereinigt und 7 µg DNA wurde zur Umsetzung des für Riboflavin prototrophen Stamms S. cerevisiae JC2a (MA-Tαhis3Δ1 leu2-3 leu 2-112 ura3-52) eingesetzt. Die Transformation wurde in ein uracylfreies Medium, SC-ura, zur Selektion der Transformanden, aus denen das bei der Umsetzung eingesetzte DNA-Fragment bestanden hätte, gesäht.

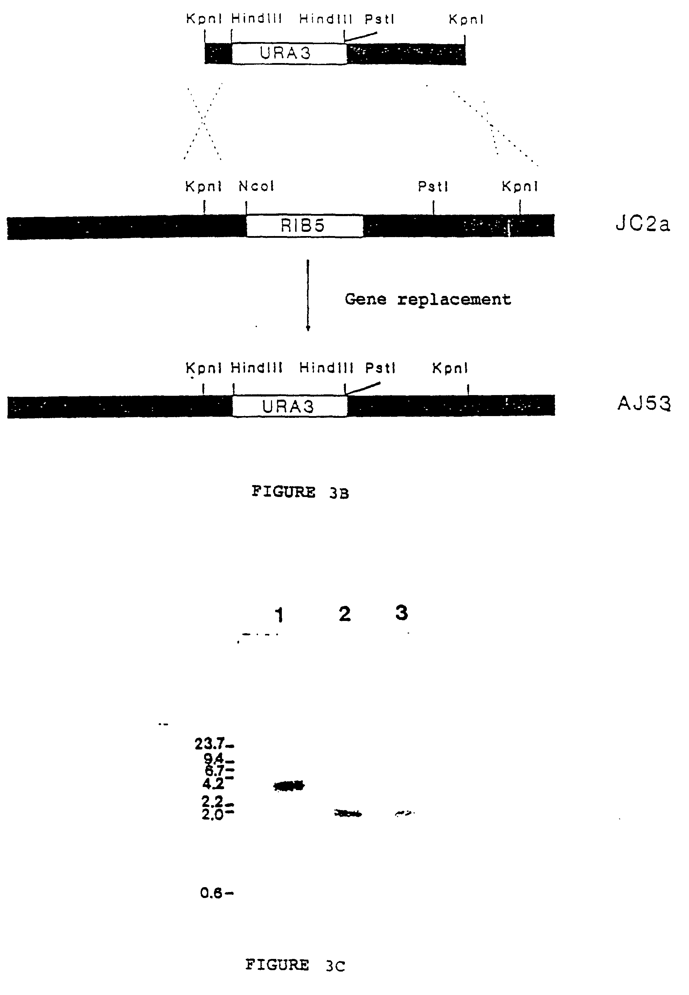

Zwei der 17 erhaltenen Ura+ Transformanden stellen außerdem den Rib-Phenotyp dar, was zeigt, daß dort das native RIB 5-Gen durch die in vitro- Deletion ersetzt worden ist. Die in diesen Stämmen ausgelöste Disruption des RIB5-Gens, AJ53 (MATαhis3Δ1 leu2-3 leu 2-112 rib5-Δ11::URA3 ura3-52) wurde anschließend durch Southern-Hybridisierung (Figur 3C) und Vervollständigung mit rib5-Mutanten bestätigt.

### Beispiel 3

### Aufbau von Plasmiden und S. cerevisiae-Stämmen mit Transkriptionsmodifikatoren des RIB5-Gens

### 3.A. Aufbau des Plasmids pJR376

Wie bereits erwähnt, kann es sehr nützlich sein, den Promotor des RIB5-Gens durch Promotoren zu ersetzen, die eine größere Expression zum Aufbau dieses biosynthetischen Gens ermöglichen. Die diese Konstruktionen enthaltenen Plasmide können zur Herstellung von Hefestämmen verwendet werden, die höhere Mengen Riboflavin produzieren.

Dadurch, daß man die Nukeotidsequenz des RIB5-Gens sowie der Promotor des stark in Hefen exprimierbaren, die 3-Phoshoglyceratkinase, PGK, codierenden Aufbaugens, kannte, wurde eine Konstruktion entworfen, bei der der Kodierbereich des RIB5-Gens vom PGK-Genpromotor durch Polymerase-Kettenreaktion, nach der von Yon et al. (Nucleic Acids Res. 17:4895, 1989) beschriebenen Methode kontrolliert wird.

Zur Herstellung eines die Fusion PGK-RIB5 enthaltenen DNA-Fragments wurden 4 Nukleotide in einem automatischen DNA-Synthesizer von Applied Biosystems Model 380 A (Foster City, CA, USA) synthetisiert und anschließend durch Gelelektrophorese wie vom Hersteller beschrieben gereinigt. Die Nukleotidsequenzen dieser Primer und ihre Beschreibung ist wie folgt:

Primer A ist homolog (Nukleotide 8 bis 28) der Nukleotidsequenz der Kodierkette des PGK-Gens (Nukleotide -580 bis -620, numeriert nach Hitzeman et al. Nucleic Acids Res. 10:7791, 1982). Dieser Primer besitzt außerdem an seinem 5' Ende die Sequenz der Erkennungsbasen des Restriktionsenzyms Ncol (Nukleotide 1 bis 6) und BAmHI (Nukleotide 5 bis 10). Primer B ist komplementär zur Nukleotidsequenz der Kodierkette des PGK-Gens (Nukleotide -17 bis +3). Primer C ist homolog in seinen Nukeotiden 1 bis 15 der Nukleotidsequenz der Kodierkette des PGK-Gens (Nukleotide -12 bis +3) und der Nukelotidsequenz der Kodierkette des RIB5-Gens (Nukleotide +1 bis +15, numeriert wie oben angegeben). Primer D ist komplementär zur Nukleotidsequenz der Kodierkette des RIB5-Gens (Nukleotide +738 bis +755).

Es wurde eine 100 µl Reaktionsmischung aus 10mM Tris.HCl, pH=7,3; 50mM KCl, 2mM MgCl₂, 80nM jedes Desoxynukleotidtriphosphats, 50 pmol jedes Primers [A und B, C und D, oder A und D), 50 ng DNA-Form wie nachfolgend beschrieben und 0,5 Einheiten Taq Polymerase, hergestellt. Die Reaktionen wurden für 30 Zyklen in einem DNA Thermal Cycler (Perkin-Elmer Cetus, Norwalk, CT, USA) durchgeführt. Jeder Zyklus umfaßte eine Denaturierungsstufe durch eine einminütige Wäremeinwirkung bei 94°C, gefolgt von einem Reannealing der Primer an die DNA bei 55°C während 2,5 min und einer Kettenverlängerungsstufe durch die Taq Polymerase während 2,5 min bei 72°C. Auf die Reaktionsmischungen wurde ein Tropfen Mineralöl gegeben, das vor den Restriktionsenzymreaktionen einmal mit Chloroform extrahiert worden war.

In einer ersten Reaktionsmischung, welche die Primer A und B sowie die genomische DNA des Wildtyp Stamms von S. cerevisiae X2180-1A (dieser Stamm kann beim Yeast Genetic Stock Center, University of California, Berkely, CA, USA, erhalten werden) enthielt und nach dem von Philipsen et al. (Guide to Yeast Genetics and Molecular Biology, Methods Enzymol., 194:169, 1991) beschriebenen Verfahren hergestellt worden war, wurde ein DNA-Fragment vervielfältigt, das den PGK-Genpromotor (nt -580 bis +3) enthielt und außerdem am distalen 5'-Ende Erkennungssequenzen für *Ncol* und *BamHI* besaß.

In einer zweiten, die Primer C und D sowie die DNA des Plasmids pJR235 enthaltenden Reaktionsmischung, wurde der Kodierbereich des RIB5-Gens (nt +1 bis +755) vervielfältigt, der außerdem an dem Nukleotid +1 entsprechenden Ende eine dem PGK-Gen entsprechende Sequenz (nt -12 bis +3) besaß.

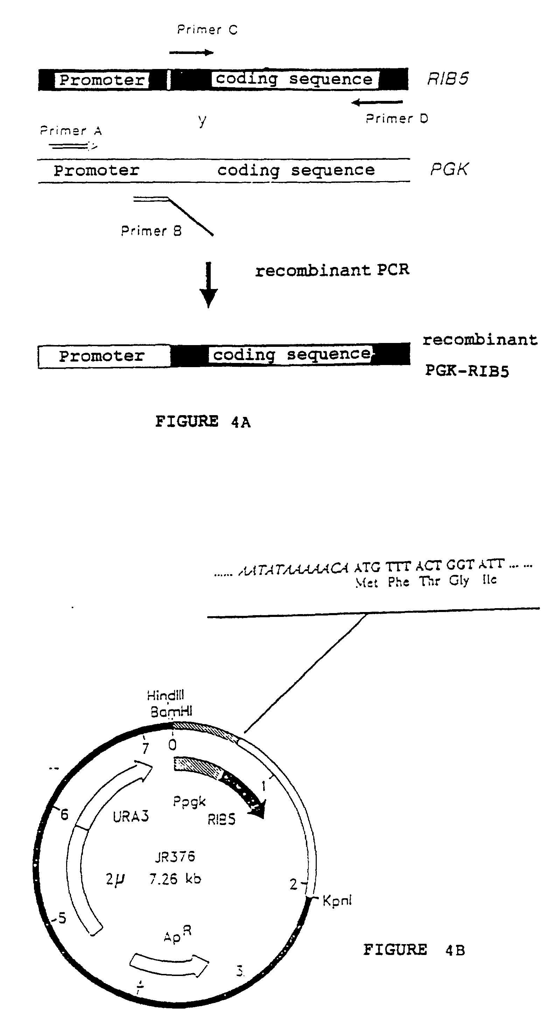

In einer dritten Reaktionsmischung, welche die Primer A und D sowie gereinigte DNA, entsprechend den zwei, in den zwei vorangehenden Reaktionen vervielfältigten Fragmenten (250 ng jedes Fragments), enthielt, wurde ein DNA-Fragment vervielfältigt, in dem die Sequenzen des PGK-Genpromotors (nt -580 bis +3) mit dem Kodierbereich des RIB 5-Gens (nt +1 bis +755), so wie in Figur 4A skizziert, ligiert sind.

Dieses Reaktionsprodukt wurde im Agarosegel gereinigt, mit BamHI verdaut und im mit BamHI und SmaI verdauten YEp352-Vektor kloniert, wodurch das Plasmid pJR376 entstand (Figur 4B). Die richtige Nukleotidsequenz der genetischen Fusion PGK-RIB5, die nach den Vervielfältigungsreaktionen fehlerfrei ist, wurde anschließend durch Sequenzierung bestätigt.

### Beispiel 4

### Charakterisierung der Riboflavinsynthetase

### 4A. Expression des RIB5-Proteins in E. coli

Zur Expression der Riboflavinsynthetase in E. coli wurde der Vektor pTrc99A verwendet, der den synthetischen trc-Promotor trägt (Amann et al Gene 69:301, 1988. Dieser Vektor kann bei Pharmacia LKB Biotechnology, Uppsala, Schweden erhalten werden). Um die Kodiersequenz del RIB5-Gens unter die Kontrolle des trc-Promotors zu stellen, wurde vorher durch Mutagenese nach dem von Kadowaki et al (Gene, 76:161, 1989) beschriebenen Verfahren eine Restriktionsstelle *Ncol* um das Initiationscodon 5'-ATG-3' der Kodiersequenz des RIB5-Gens herum geschaffen. Hierzu wurden als Primer zwei Oligonukleotide verwendet: E zur Schaffung der Restriktionsstelle Ncol, und D, der dem vorher in Beispiel 3A beschriebenen entspricht. Diese Oligonukleotiden wurden in einem automatischen DNA-Synthesizer von Applied Biosystems, Model 380 A, (Foster City, CA, USA), hergestellt und seine Sequenzen sind wie folgt:

In einer Polymerase-Kettenreaktion (PCR) mit einem Volumen von 100 µl, enthaltend 10mM Tris HCl, pH=7,3; 50 mM KCl, 2mM MgCl₂, je 80nM der einzelnen Desoxynukleotidtriphosphate, jeweils 50 pmol der Primer D und E, 50 ng DNA des Plasmids pJR235 und 0,5 Einheiten der Taq Polymerase (Kit GeneAmp, Perkin-Elmer Cetus), wurde ein DNA-Fragment vervielfältigt, das die modifizierte Sequenz des RIB5-Gens mit einer Restriktionsstelle Ncol im Initiationscodon 5'-ATG-3' enthält. Das Fragment wurde in Agarosegel gereinigt, mit *NcoI* verdaut, an den vorher mit NcoI-SmaI verdauten pTrc99A-Vektor ligiert und in E.coli JM101 transformiert. Ein rekombinantes Plasmid wurde selektiert und pJR380 benannt.

Zur Expression des RIB5-Gens von S. cerevisiae in E. coli ließ man mit dem Plasmid pJR380 transformierte E. coli JM101-Zellen 16 Stunden im NZY^{Amp100}-Medium wachsen (1 % Caseinhydrolysat, 0,5 % NaCl, 0,5 % Hefeextrakt, 0,2 % MgSO₄ und 100 µg/ml Ampicillin) und induzierte sie 2 Stunden mit 1 mM Isopropyl-β-D-thiogalactopyranosid (IPTG). Die mit dem Expressionsplasmid pJR380 transformierten Zellen wuchsen langsamer als die mit dem pTrc99A-Vektor transformierten. Nach Induktion mit IPTG wuchsen die das Expressionsplasmid pJR380 enthaltenden Zellen bis zum Stillstand. Eine mikroskopische Prüfung dieser Transformanden zeigte das Auftreten von verlängerten Zellen, die lichtbrechende Granula, Einschlußkörper genannt, in den Zellpolen enthielten, und die typisch für die Morphologie von Zellen sind, die große Mengen an unlöslichem Fremdprotein enthalten (Sharma, S.K., 1986. Separation Sci. Technol. 21:701-726, 1986).

Anschließend wurden die Zellen durch Zentrifugieren geerntet, in Lysis Puffer erneut suspendiert, durch Ultraschall aufgeschlossen, die Zellextrakte in lösliche und unlösliche Fraktionen durch 15 minütiges Zentrifugieren bei 12.000 U/min und 4°C nach dem von Sambrook et al. beschriebenen Verfahren (1989, Molecular cloning. A laboratory manual. Cold Spring Harbor, New York, Cold Spring Harbor Laboratory Press) getrennt.

Diese Fraktionen wurde durch Elektophorese unter denaturierenden Bedingungen analysiert und die Gele mit Coomassie Blau gefärbt. Während bei den löslichen Fraktionen keine zusätzlichen Banden zu den normalen auftraten, zeigten die unlöslichen Fraktionen der mit dem Expressionsplasmid pJR380 ungesetzten Zellen eine intensive Bande eines Molekulargewichts von ca. 24 kDa (Fig.5), das dem abgeleiteten Gewicht des Kodierbereichs des klonierten RIB5-Gens entspricht. Diese Ergebnisse zeigen, daß das RIB5-Protein in E. coli in hohem Grad exprimiert werden kann, auch wenn der größte Teil des Proteins in unlöslicher, enzymatisch inaktiver Form vorliegt und zu seiner Extraktion die Verwendung von Detergentien oder denaturierenden Agentien erforderlich ist.

### 4.B. Herstellung und Reinigung von polyklonalen Antikörpern anti-Rib5

Das unlösliche, in E. coli JM101 exprimierte Rib5-Protein ist hervorragend geeignet, um als Immunogen bei der Herstellung von polyklonalen Antikörpern, so wie im folgenden beschrieben, eingesetzt zu werden. Der unlösliche, das Rib5-Protein enthaltende Niederschlag aus 250 ml mit dem Expressionsplasmid pJR380 transformierten, mit IPTG induzierten, wie in Beispiel 4 A beschrieben hergestellten Zellen, wurde mit 20 ml destilliertem Wasser gewaschen und in 4 ml 0,1M Tris.HCl, pH=8,5, und 3M Harnstoff solubilisiert. Die Mischung wurde 15 Minuten bei 4°C und 12.000 U/min zentrifugiert und der Harnstoff durch Dialyse in 0,1M Tris.HCl entfernt. Es wurdem insgesamt 5 mg Protein 99 %ig reines Rib5-Protein, photometrisch bestimmt (Fig. 5A), erhalten.

Neuseeland-Kaninchen, denen, um Präimmun-Serum zu erhalten, vorher Blut entnommen wurde, wurden subkutan 100 µg Rib5-Protein in 0,5 ml 100mM Tris.HCl, pH=8,5, und 2 % bei 100 °C 3 min. denaturiertes und mit vollständigem Freund-Adjuvans (Hoechst-Behring) emulgiertes SDS, gespritzt. Anschließend wurden die Kaninchen alle 2 Wochen mit einer Auffrischungsdosis inokuliert und 6 Wochen nach der Anfangsimmunisierung wurde ihnen Blut entnommen, um Antiserum zu erhalten.

Die Antikörper wurden durch Affinitätschromatographie in einer Säule, welche das in E. coli JM101 exprimierte Rib5-Protein gebunden an eine Affi-Gel 10-Matrix (BioRad, Richmond, CA) enthielt, nach den Hinweisen der Hersteller gereinigt. Die Säule wurde gründlich mit Staph-Puffer (1,6 µM NaH₂PO₄, 1,1 µM Na₂HPO₄·7H₂O 1 % Triton X100, 0,1 % SDS, 0,1 % NaN₃, 0,1 M NaCl, 0,5 % Desoxynatriumcholat) gewaschen und anschließend mit 20 Volumen 0,2 M Glycin pH 2.2 eluiert und das Eluat sofort mit 1/9 Volumen 1M Tris.HCl, pH=8,8, neutralisiert und bei 4°C in Anwesenheit von 0,1 % Azid aufbewahrt. Als diese Antikörper in Western Blots der Gesamtproteine von E. coli nachgewiesen waren, wurde eine Reaktion mit Polypeptiden der geeigneten Größe beobachtet. Wie bei den mit Coomassie Blau gefärbten Gelen, lag der größte Teil des durch Western Blotting nachgewiesenen Rib5-Proteins in unlöslicher Form vor (Figur 5B).

### 4.C. Reinigung der Riboflavinsynthetase con S. cerevisiae

Die Riboflavinsynthetase katalysiert die Bildung eines Riboflavin- und eines 5-Amino-6-ribitylamino-2,4(1H,3H)-pyrimidinedionmoleküls aus zwei Molekülen 6,7-Dimethyl-8-ribityl-lumazin. Das Enzym wurde in mehreren Mikroorganismen und Pflanzen gefunden und es wurden bereits teilgereinigte Zubereitungen von Ashbya gossypii, Escherichia coli und Spinat hergestellt. Die Riboflavinsynthetase aus Hefe wurde bereits rund 5000 fach gereinigt, obwohl das Reinigungsverfahren sehr aufwendig und mühsam ist (Plaut, G.W.E. et al., Biochemistry 9:771-785, 1970)

Der Gehalt an Riboflavinsynthetase in Zellextrakten von niederen Mikroorganismen, einschließlich hochflavinogener Ascomyceten ist gering, was ihre Reinigung und Charakterisierung schwierig macht. Daher ist es wünschenswert über ein einfaches und reproduzierbares Reinigungsverfahren für das Enzym sowie alternative Nachweismethoden zu verfügen, die nicht auf seiner enzymatischen Aktivität beruhen.

Zur Reinigung der Riboflavinsynthetase von S. cerevisiae wurden Zellextrakte nach dem Verfahren von Michaeli et al. (EMBO *J.* 10:3045, 1989) aus 250 ml einer Kultur von Zellen des Wildtyp-Stammes X2180-la mit einer OD₆₀₀=1,5-2,0 im YPD-Medium hergestellt. 1ml des erhaltenen Zellextrakts (≃ 5 mg Protein/ml) wurde durch FPLC-Chromatograhie unter Verwendung einer vorher mit 20mM Tris.HCl-Puffer, pH=7,5, äquilibrierten Ionenaustauschsäule MonoQ HR-5/5 (Pharmacia) fraktioniert. Die zurückgehaltenen Proteine wurden in einem kontinuierlichen Gradient von 0 bis 0,5M NaCl eluiert und die 1 ml Fraktionen durch Western blotting mit nach dem Verfahren aus Beispiel 4B erhaltenen anti-Rib5-Antikörpern analysiert. Die anti-Rib5-Antikörper reagierten spezifisch mit einem Polypeptid von ca. 24 kDa, das in der Fraktion 22, entsprechend einer NaCl Konzentration von 250 mM, eluierte. Das Molekulargewicht dieses Polypeptids stimmt mit dem abgeleiteten Gewicht der Nukleotidsequenz des Kodierbereichs des klonierten Gens überein.

Die Fraktionen vor der Ionenaustauchchromatographie wurden ebenfalls auf Riboflavinsynthetaseaktivität nach dem von Bacher et al beschiebenen Verfahren (Methods Enzymol. 122:192, 1986) geprüft. Konkret enthielten die Prüfmischungen 50mM Kaliumphosphat pH=7, 10mM NaHSO₄, 0,6 mM 6,7-Dimethyl-8-ribityllumazin synthetisiert nach dem von Plaut und Harvey beschriebenen Verfahren (Methods Enzymol. 18B:515-538, 1971) und 150 µl der zu prüfenden Fraktion, in eimem Gesamtvolumen von 1 ml. Die Reaktionsmischungen wurden 1 Stunde in der Dunkelheit bei 37°C inkubiert. Am Anfang und am Ende der Inkubationszeit wurden Proben entnommen und das Protein durch Zugabe von 15 % Trichloressigsäure gefällt.

Die Menge an synthetisiertem Riboflavin wurde aus der Zunahme der optischen Dichte bei 470 nm errechnet (molare Extinktion des Riboflavins bei 470 nm = 9300) und die spezifische Aktivität in nmol pro Stunde und mg Protein gebildetem Riboflavin ausgedrückt. Die Ergebnisse zeigen, daß die Riboflavinsynthetaseaktivität ein Maximum in der bei einer NaCl-Konzentration von 250 mM eluierten Fraktion 22 zeigte, wobei es sich hier um dieselbe Fraktion handelt, die im Polypeptid festgestellt worden war, das mit den anti-Rib5-Antikörpern reagiert hatte.

Wenn die in der Fraktion 22 vorliegenden Proteine elektophoretisch unter denaturierenden Bedingungen analysiert werden und die Gele mit Silber gefärbt werden (Morrisey, Anal. Biochem., 117:507, 1981), zeigt sich eine einzige größere Bande, die mehr als 99 % des Gesamtproteins der Fraktion ausmacht, und die ein Molekulargewicht von ca. 24 kDa aufweist, was mit dem abgeleiteten Molekulargewicht der Nukleotidsequenz des Kodierbereichs des klonierten Gens übereinstimmt (Figur 6B).

### 4.D. Charakterisierung der Riboflavinsynthetase von S. cerevisiae

### 4.D.1 Sequenzierung des aminoterminalen Endes der Riboflavinsynthetase

In eukaryontischen Organismen sind nach der wirksamen Translation der mRNA in einem Polypeptid häufig spätere Modifikationen zur Herstellung eines reifen Polypeptids erforderlich. Um festzustellen, ob das Rib5-Protein in seinem aminoterminalen Ende irgendeine Änderung aufweist und um nachzuweisen, daß das von der Erfindung vorgeschlagene Startcodon (erstes ATG in der Phase im ORF) tatsächlich mit dem für die Synthese des Rib5-Proteins verwendeten Startcodons (erstes ATG in der Phase im ORF) übereinstimmt, wurde das aminoterminale Ende des Rib5-Proteins sequenziert. 5 µg des durch Ionenaustauchchromatographie nach dem Verfahren gemäß Beispiel 4C gereinigten Rib5-Proteins wurden einer Elektrophorese bei denaturierenden Bedingungen in einem 14 % Acrylamid enthaltenden Gel unterworfen und auf eine Difluorpolyvinylidenmembran, PVDF, (Inmobilon-P, Millipore Corporation, Bedford, MA) nach dem Verfahren von Matsudaira (J. Biol. Chem. 262:10035, 1987) übertragen.

Nach der Übertragung wurde die Membran mit 20 % Ethanol benetzt, und die dem Rib5-Protein entsprechende, nach Durchleuchtung sichtbar gemachte Bande ausgeschnitten und in einem Protein-Sequencer von Applied Biosystems Model 470, Gas-Phase Sequencer, (Foster City, Ca, USA) sequenziert. Die Sequenz der ersten acht analysierten Aminosäurereste: NH₂-Met-Phe-Thr-Gly-Ile-Val-Glu-Cys-...-COOH ist identisch mit der aus der Nukleotidsequenz des klonierten Gens abgeleiteten Aminosäuresequenz, was darauf hinweist, daß das im Rahmen der Erfindung vorgeschlagene Startcodon (erstes ATG in der Phase im ORF) das richtige ist, und daß das Rib5-Protein, die Riboflavinsynthetase, keine Änderung an seinem aminoterminalen Ende aufweist.

### 4.D.2 Bestimmung des Molekulargewichts der Riboflavinsynthetase unter nativen Bedingungen

Um festzustellen, ob die Riboflavinsynthetase unter nativen Bedingungen in monomerer oder multimerer Form vorliegt, wurde das Molekulargewicht des Rib5-Proteins unter nativen Bedingungen bestimmt. Nach Beispiel 4C gereinigtes Rib5-Protein (0,5 µg) wurde in einem Gel eines Polyacrylamid-Gradienten von 4 % bis 20 % (Bio-Rad, Richmond, CA) für 12 h bei 25 mA und 4°C in Tris-Glycin-Puffer (25mM Tris, 150mM Glycin) zusammen mit nativen Molekulargewichtsmarkern aus Tiroglobulin (669 kDa), Ferritin (440 kDa), Catalase (232 kDa), Lactatdehydrogenase (140 kDa) und Rinderserumalbumin (67 kDa) analysiert.

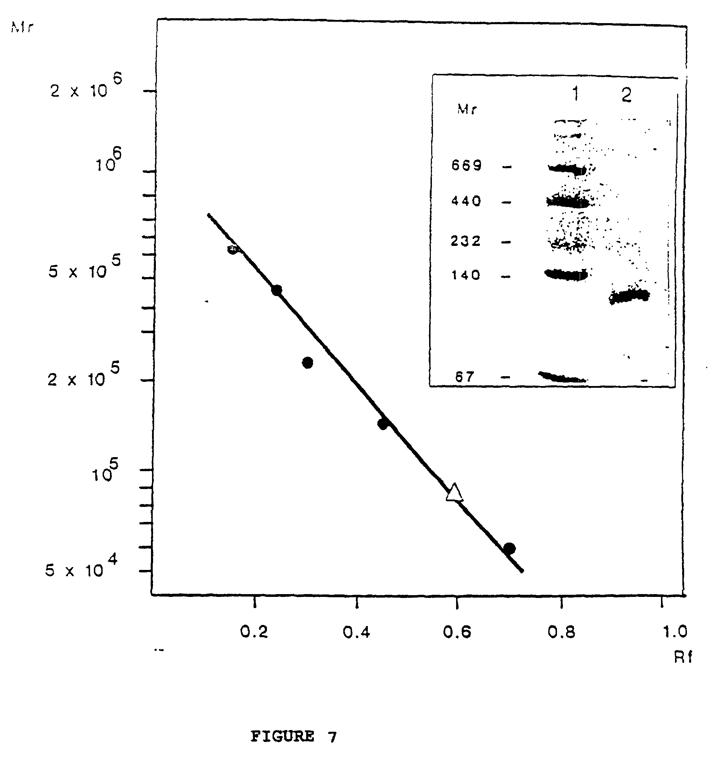

Nach Termination der Elektrophorese wurde das Gel mit Silber gefärbt und das Molekulargewicht des Rib5-Proteins unter nativen Bedingungen nach dem von Andersen et al. (FEBSLett. 20:199, 1971) beschriebenen Verfahren geschätzt. Die Ergebnisse (Figur 7) zeigen, daß das Rib5-Protein unter nicht denaturierten Bedingungen ein Molekulargewicht von ca. 72 kDa haben, was bedeutet, daß die Riboflavinsynthetase im nativen Zustand ein Multimer aus drei identischen Rib5-Polypeptid-Untereinheiten besteht. Diese Ergebnisse wurden durch Cross-Linking Versuche in Anwesenheit von Dimethylsuberimidat nach dem von Davies und Stark beschriebenen Verfahren (Proc.Natl. Acad. Sci. USA 66:651, 1970) bestätigt.

### Beispiel 5

### Herstellungsrate von Riboflavinsynthetase

Die Herstellung von Riboflavinsynthetase wurde bei einem Wildtyp Stamm von S. cerevisiae und Trägerstämmen verschiedener Modifikationen des RIB5-Gens, die für die Riboflavinsynthetase codieren, bestimmt. Proteinextrakte von Kulturen in der stationären Phase der wilden Stämme X2180-1A, Träger einer kompletten Deletion des RIB5-Gens, AJ113, Transformand AJ53, Träger des das RIB5-Gen enthaltenen Multikopienplasmids pJR235, sowie AJ112, Transformand AJ53, Träger des die genetische Fusion PGK-RIB5 enthaltenen Multikopien Plasmids pJR376, wurden auf ihre Riboflavinsynthetaseaktivität nach dem Verfahren gemäß Beispiel 4C analysiert.Es wurden die nachfolgenden, in Tabelle I aufgeführten Ergebnisse erhalten.

**Tabelle I**

| Stamm | Spezifische Riboflavinsynthetaseaktivität (nMol Riboflavin/mg Protein) |
|---|---|
| X2180-1A | 5,59 |
| AJ53 | ---- |
| AJ113 | 71,6 |
| AJ112 | 284,01 |

Zu Tabelle I wäre kurz zu sagen, daß die Anwesenheit des RIB5-Gens in einem Multikopienplasmid eine mindestens 20 fache Erhöhung der Riboflavinsynthetaseaktivität im Wildtyp Stamm X2180-1A voraussetzt. Diese Aktivität kann noch zusätzlich durch die Verwendung eines stark exprimierenden Promotors, z.B. den Promotor des PGK-Gens, erhöht werden, was dazu führt, daß der Stamm AJ112 eine 80 fach höhere Riboflavinsynthetaseaktivität hat, als der Wildtyp Stamm.
Erklärung der Legenden der Figuren

### Figur 1A:

(a) Plasmid
(b) Komplementation der rib5-10-Mutation

### Figur 3A:

(a) Verdau mit Ncol
(b) Behandlung mit dem Klenow-Fragment
(c) Verdau mit BamHI
(d) Reinigung des Fragments BamHI-Nco1IK 1,6 Kb.
(e) Verdau mit KpnI und PstI
(f) Ligierung
(g) Verdau mit KpnI und HindIII
(h) Verdau mit BamHI und SmaI
(i) Verdau mit HindIII

### Figur 6A:

(a) Spezifische Aktivität (nMol B2/mg)
(b) Fraktionsnummer
(c) NaCl (M)

### Hinterlegung von Mikroorganismen

Die Transformanden von E. coli JM101/pJR235 wurden am 17. März 1992 bei National Collections of Industrial and Marine Bacteria Limited (NCIMB in Aberdeen (Schottland, Großbritanien) unter der NCIMB-Nummer 40480 hinterlegt.

## Patentansprüche

1. Verwendung des Riboflavinsynthetasegens aus Saccharomyces cerevisiae zur Herstellung von Expressionsvektoren für die Steigerung der Riboflavinsynthese in damit transformierten Wirtszellen.

2. Verfahren zur rekombinanten Herstellung von Riboflavin durch Transformation einer Wirtszelle mit einem Expressionsvektor, der zur Expression der Riboflavinsynthetase von S. cerevisiae befähigt ist, **dadurch gekennzeichnet, daß** in dem geeigneten Expressionsvektor
(a) eine, die Riboflavinsynthetaseaktivität codierende DNA-Verbindung, oder
(b) eine zur obigen Verbindung homologe Sequenz, die diese Aktivität exprimiert und eine genetische Variante derselben darstellt, oder
(c) ein die Riboflavinsynthetaseaktivität codierendes Fragment derselben, kloniert wird.

## Claims

1. The use of the riboflavin synthetase gene from Saccharomyces cerevisiae for preparing expression vectors for increasing riboflavin synthesis in host cells transformed therewith.

2. A process for the recombinant preparation of riboflavin by transforming a host cell with an expression vector capable of expressing the riboflavin synthetase from Saccharomyces cerevisiae, which comprises cloning in the suitable expression vector
(a) a DNA compound encoding riboflavin synthetase activity or
(b) a sequence which is homologous to the above compound, expresses this activity and represents a genetic variant thereof or
(c) a fragment thereof encoding riboflavin synthetase activity.

## Revendications

1. Utilisation du gène de riboflavine synthétase de Saccharomyces cerevisiae pour la préparation de vecteurs d'expression pour augmenter la synthèse de riboflavine dans des cellules hôtes ainsi transformées.

2. Procédé pour la préparation recombinante de riboflavine par transformation d'une cellule hôte avec un vecteur d'expression qui est apte à l'expression de la riboflavine synthétase de S. cerevisiae, **caractérisé par le fait qu'**on clone dans le vecteur d'expression approprié
(a) un composé d'ADN codant pour l'activité de riboflavine synthétase, ou
(b) une séquence homologue pour le composé susdit, qui exprime cette activité et représente une variante génétique de celui-ci, ou
(c) un fragment de celui-ci codant pour l'activité de riboflavine synthétase.


### SEQUENZPROTOKOLL

(1) ALGEMEINE INFORMATION:
   (i) ANMELDER:
      (A) NAME: BASF Aktiengesellschaft
      (B) STRASSE: Carl-Bosch-Strasse 38
      (C) ORT: Ludwigshafen
      (E) LAND: Bundesrepublik Deutschland
      (F) POSTLEITZAHL: D-6700
      (G) TELEPHON: 0621/6048526
      (H) TELEFAX: 0621/6043123
      (I) TELEX: 1762175170
   (ii) ANMELDETITEL: DNA-Verbindungen und rekombinante, die Riboflavinsynthetaseaktivitaet von S. cerevisiae codierende DNA-Expressionsvektoren
   (iii) ANZAHL DER SEQUENZEN: 8
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: Patent In Release #1.0, Version #1.25 (EPA)
   (2) INFORMATION ZU SEQ ID NO: 1:
      (i) SEQUENZ CHARAKTERISTIKA:
         (A) LÄNGE: 1439 Basenpaare
         (B) ART: Nukleinsäure
         (C) STRANGFORM: Doppel
         (D) TOPOLOGIE: linear
      (ii) ART DES MOLEKÜLS: DNS (genomisch)
      (iii) HYPOTHETISCH: NEIN
      (iii) ANTISENSE: NEIN
      (vi) URSPRÜNLICHE HERKUNFT:
         (A) ORGANISMUS: Saccharomyces cerevisiae
      (ix) MERKMALE:
         (A) NAME/SCHLÜSSEL: CDS
         (B) LAGE: 1..717
      (xi) SEQUENZBESCHREIBUNG: SEQ ID NO: 1:
   ATG TTT ACT GGT ATT GTA GAA TGC ATG GGG ACT GTT TTG GAA AAC AAC       48
   Met Phe Thr Gly Ile Val Glu Cys Met Gly Thr Val Leu Glu Asn Asn
    1               5                  10                  15
   CCA TAT GAT GAC TCT GAA AGT GGA GGT CAA GGA GTT TCT ATT ACT ATT       96
   Pro Tyr Asp Asp Ser Glu Ser Gly Gly Gln Gly Val Ser Ile Thr Ile
                20                  25                  30
   GGC AAT GCG GGG AGT ATT CTC ACC GAT TGT CAC GTT GGA GAT TCA ATA      144
   Gly Asn Ala Gly Ser Ile Leu Thr Asp Cys His Val Gly Asp Ser Ile
            35                  40                  45
   GCC GTA AAT GGG GTA TGC CTT ACT GTG ACC GAG TTT AAT AAC GAC TCC      192
   Ala Val Asn Gly Val Cys Leu Thr Val Thr Glu Phe Asn Asn Asp Ser
        50                  55                  60
   TTC AAA GTT GGG ATA TCA CCA GAA ACT ATA AAA CGA AGT AAT GTC GCT      240
   Phe Lys Val Gly Ile Ser Pro Glu Thr Ile Lys Arg Ser Asn Val Ala
    65                  70                  75                  80
   TCC TGG ATT CAA GGC ACC CAG GTC AAC TTG GAG AGA GCG GTA TCT CAA      288
   Ser Trp Ile Gln Gly Thr Gln Val Asn Leu Glu Arg Ala Val Ser Gln
                    85                  90                  95
   GAC GTT AGG TTC GGT GGT CAT TAT GTA CAG GGT CAC GTA GAC ACT GTT      336
   Asp Val Arg Phe Gly Gly His Tyr Val Gln Gly His Val Asp Thr Val
               100                 105                 110
   GCT AAT ATT GTC TCA AGA AGA CCT GAG GGG AAT TCA ATT ATT TTT GGG      384
   Ala Asn Ile Val Ser Arg Arg Pro Glu Gly Asn Ser Ile Ile Phe Gly
           115                 120                 125
   TTT CAG TTA AGA GAT CAA GAG TAC TTT AAA TAC ATA GTA GAA AAG GGA      432
   Phe Gln Leu Arg Asp Gln Glu Tyr Phe Lys Tyr Ile Val Glu Lys Gly
       130                 135                 140
   TTC ATT TGT ATA GAT GGA ACT TCC TTG ACC ATA ATC AAG GTT GAC CCA      480
   Phe Ile Cys Ile Asp Gly Thr Ser Leu Thr Ile Ile Lys Val Asp Pro
   145                 150                 155                 160
   CTT TCG CAA GGT GGA GCC TTC TAT ATT AGT ATG ATA AAG CAC ACC CAA      528
   Leu Ser Gln Gly Gly Ala Phe Tyr Ile Ser Met Ile Lys His Thr Gln
                   165                 170                 175
   GAC AAT GTT ATC ATG CCT TTG AAG AAA ATT GGC GAC GAG GTT AAT ATT      576
   Asp Asn Val Ile Met Pro Leu Lys Lys Ile Gly Asp Glu Val Asn Ile
               180                 185                 190
   GAA GTA GAT TTG ACT GGG AAG ATT ATT GAG AAG CAA ATT CTA TTA ACG      624
   Glu Val Asp Leu Thr Gly Lys Ile Ile Glu Lys Gln Ile Leu Leu Thr
           195                 200                 205
   TTG GAA AAC CAA ATA TCA AAG AAA GAT AGT ACT TTG AAT ACT ATG ATC       672
   Leu Glu Asn Gln Ile Ser Lys Lys Asp Ser Thr Leu Asn Thr Met Ile
       210                 215                 220
   TCA AAC ATT ATC GAG GAG AAG GTT AGA AAC TAC CTA AAT AAA TAAAATAAAA    724
   Ser Asn Ile Ile Glu Glu Lys Val Arg Asn Tyr Leu Asn Lys
   225                 230                 235
   AATTCGTCAT AAATAGGTTT CATAAACGCA TTTTTGAAGT GATCTCTCCA TCTGCTGCAA     784
   AAATAGTCTT GTAAATGTGT GATAATGATG AGATTAGAAC TTGGTACTGT AGCTAAGGTA     844
   CTTAGGCAAC TTCTCTTCAT TATGTATTAC CATGTTCTGT TGGTTCACGT GACATGCAAA     904
   GGTATAACTT TTTCCGTACA AATTATTCTC TCCACCTTTT TTGTGTTGTT GTGAAAGAAC     964
   TTTCCAATTA TTGCCACTGT AACCAACCGT CCCTAAATAC CAACGACACA AAAACTGCTG    1024
   CTACTATGGG TGGTAACGTT CTCCCCATTC ATTATGACCC CAAAACTGTT AAACAATTAA    1084
   CTAAAGAAGT ATGTAAAATT CAAGAATATA TCTGGCCCTG TCTTGTTCAC CCCAGAATAA    1144
   ATCGCTAACA CAAGTCTAAT TAACCTTCTT TCTCATTCGT AGATTACTGT TGCATCATGT    1204
   ATAGGAGCTG CCAAGGTGCT CTCTTCAGTC TTGCCTCAGC GCTGCTGTTG AGAAGATTTT    1264
   CATCAGTATA TAGAAATGTC AGAACTCAAG TGCGTGTTTT TTATCATTGT TCTTGGATTT    1324
   CAATGGGTGC TGTCTTCAGA GCTGATAAGC AATTGCTCAA GTTCCAGACA AATTATTATC    1384
   GTGAGGAACA GAAAAGAAGA GAAAAGATTA TGGATGAAGC TGCTGAGAGA GGCCT         1439
(2) INFORMATION ZU SEQ ID NO: 2:
   (i) SEQUENZ CHARAKTERISTIKA:
      (A) LÄNGE: 238 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Phe Thr Gly Ile Val Glu Cys Met Gly Thr Val Leu Glu Asn Asn
     1               5                  10                  15
   Pro Tyr Asp Asp Ser Glu Ser Gly Gly Gln Gly Val Ser Ile Thr Ile
               20                  25                  30
   Gly Asn Ala Gly Ser Ile Leu Thr Asp Cys His Val Gly Asp Ser Ile
           35                  40                  45
   Ala Val Asn Gly Val Cys Leu Thr Val Thr Glu Phe Asn Asn Asp Ser
       50                  55                  60
   Phe Lys Val Gly Ile Ser Pro Glu Thr Ile Lys Arg Ser Asn Val Ala
    65                  70                  75                  80
   Ser Trp Ile Gln Gly Thr Gln Val Asn Leu Glu Arg Ala Val Ser Gln
                    85                  90                  95
   Asp Val Arg Phe Gly Gly His Tyr Val Gln Gly His Val Asp Thr Val
               100                 105                 110
   Ala Asn Ile Val Ser Arg Arg Pro Glu Gly Asn Ser Ile Ile Phe Gly
           115                 120                 125
   Phe Gln Leu Arg Asp Gln Glu Tyr Phe Lys Tyr Ile Val Glu Lys Gly
       130                 135                 140
   Phe Ile Cys Ile Asp Gly Thr Ser Leu Thr Ile Ile Lys Val Asp Pro
   145                 150                 155                 160
   Leu Ser Gln Gly Gly Ala Phe Tyr Ile Ser Met Ile Lys His Thr Gln
                   165                 170                 175
   Asp Asn Val Ile Met Pro Leu Lys Lys Ile Gly Asp Glu Val Asn Ile
               180                 185                 190
   Glu Val Asp Leu Thr Gly Lys Ile Ile Glu Lys Gln Ile Leu Leu Thr
           195                 200                 205
   Leu Glu Asn Gln Ile Ser Lys Lys Asp Ser Thr Leu Asn Thr Met Ile
       210                 215                 220
   Ser Asn Ile Ile Glu Glu Lys Val Arg Asn Tyr Leu Asn Lys
   225                 230                 235
(2) INFORMATION ZU SEQ ID NO: 3:
   (i) SEQUENZ CHARAKTERISTIKA:
      (A) LÄNGE: 155 Basenpaare
      (B) ART: Nukleinsäure
      (C) STRANGFORM: Doppel
      (D) TOPOLOGIE: linear
   (ii) ART DES MOLEKÜLS: DNS (genomisch)
   (iii) HYPOTHETISCH: NEIN
   (vi) URSPRÜNLICHE HERKUNFT:
      (A) ORGANISMUS: Saccharomyces cerevisiae
   (xi) SEQUENZBESCHREIBUNG: SEQ ID NO: 3:
   CAATTCCGTC CTTTTCTTTT TTTTCGGTAA TTTCCATGGA GGAAGGTTAT TGAACTATCT     60
   ATTAGTCATA TTCCAGATCC ATCCAAATGA TATAAAAAGA CGGTACTTCC TAACGTACAT    120
   GTCAAACCAA TTGGTGAGTA CGTAACCATA GCACA                               155
(2) INFORMATION ZU SEQ ID NO: 4:
   (i) SEQUENZ CHARAKTERISTIKA:
      (A) LÄNGE: 28 Basenpaare
      (B) ART: Nukleinsäure
      (C) STRANGFORM: Einzel
      (D) TOPOLOGIE: linear
   (ii) ART DES MOLEKÜLS: DNS (genomisch)
   (vii) UNMITTELBARE HERKUNFT:
      (B) CLON: Primer A
   (xi) SEQUENZBESCHREIBUNG: SEQ ID NO: 4:
   CCATGGATCC CTCCTTCTTG AATTGATG     28
(2) INFORMATION ZU SEQ ID NO: 5:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (vii) UNMITTELBARE HERKUNFT:
      (B) CLON: Primer B
   (xi) SEQUENZBESCHREIBUNG: SEQ ID NO: 5:
   CATTGTTTTT ATATTTGTTG 20
(2) INFORMATION ZU SEQ ID NO: 6:
   (i) SEQUENZ CHARAKTERISTIKA:
      (A) LÄNGE: 27 Basenpaare
      (B) ART: Nukleinsäure
      (C) STRANGFORM: Einzel
      (D) TOPOLOGIE: linear
   (ii) ART DES MOLEKÜLS: DNS (genomisch)
   (vii) UNMITTELBARE HERKUNFT:
      (B) CLON: Primer C
   (xi) SEQUENZBESCHREIBUNG: SEQ ID NO: 6:
   AATATAAAAA CAATGTTTAC TGGTATT 27
(2) INFORMATION ZU SEQ ID NO: 7:
   (i) SEQUENZ CHARAKTERISTIKA:
      (A) LÄNGE: 18 Basenpaare
      (B) ART: Nukleinsäure
      (C) STRANGFORM: Einzel
      (D) TOPOLOGIE: linear
   (ii) ART DES MOLEKÜLS: DNS (genomisch)
   (vii) UNMITTELBARE HERKUNFT:
      (B) CLON: Primer D
   (xi) SEQUENZBESCHREIBUNG: SEQ ID NO: 7:
   GAAACCTATT TATGACGA 18
(2) INFORMATION ZU SEQ ID NO: 8:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (vii) UNMITTELBARE HERKUNFT:
      (B) CLON: Primer E
   (xi) SEQUENZBESCHREIBUNG: SEQ ID NO: 8:
   ATAGCACCAT GGCTACTGGT                                              20